# EUROPEAN PATENT SPECIFICATION

(11) **EP 1 477 562 B1**
(45) Date of publication and mention of the grant of the patent: **07.10.2009**
(21) Application number: 02760669.8
(22) Date of filing: 20.08.2002
(51) Int. Cl.: C12N 15/31, C12N 9/62, C12N 1/15, C07K 14/38, C12R 1/69

(54) **TRANSCRIPTIONAL FACTOR, TRANSCRIPTIONAL FACTOR GENE, RECOMBINANT VECTOR CONTAINING TRANSCRIPTIONAL FACTOR GENE, KOJI-MOLD TRANSFORMED BY THE VECTOR AND METHOD OF USING KOJI-MOLD**
TRANSKRIPTIONSFAKTOR, TRANSKRIPTIONSFAKTORGEN, DAS TRANSKRIPTIONSFAKTORGEN ENTHALTENDER REKOMBINANTER VEKTOR, MIT DEM VEKTOR TRANSFORMIERTER KOJI-SCHIMMELPILZ UND VERFAHREN ZUR VERWENDUNG DES KOJI-SCHIMMELPILZES
FACTEUR TRANSCRIPTIONNEL, GENE DE FACTEUR TRANSCRIPTIONNEL VECTEUR RECOMBINANT CONTENANT UN GENE DE FACTEUR TRANSCRIPTIONNEL, MOISISSURE KOJI TRANSFORMEE PAR LE VECTEUR ET PROCEDE D'UTILISATION DE LA MOISISSURE KOJI

(30) Priority: 21.02.2002 JP 2002045090
(43) Date of publication of application: 17.11.2004
(73) Proprietor: NODA INSTITUTE FOR SCIENTIFIC RESEARCH, Noda-shi, Chiba-ken 278-0037 (JP); National Institute of Advanced Industrial Science and Technology, Tokyo 100-8921 (JP)
(72) Inventor: UMITSUKI, Genryou,, Noda-shi, Chiba 278-0037 (JP); HATAMOTO, Osamu,, Noda-shi, Chiba 278-0037 (JP); HARA, Seiichi,, Noda-shi, Chiba 278-0037 (JP); MASUDA, Tsutomu,, Noda-shi, Chiba 278-0037 (JP); SANO, Motoaki, Tsukuba Center,, Tsukuba-shi, Ibaraki 305-0046 (JP); MACHIDA, Masayuki,, Tsukuba-shi, Ibaraki 305-0046 (JP)
(74) Representative: Denison, Christopher Marcus
(86) International application number: PCT/JP2002/008376
(87) International publication number: WO 2003/070954

(56) References cited:
- GRYNBERG M. ET AL.: 'The aspergillus nudulans metE gene is regulated by a second system independent from sulphur metabolite repression' BIOCHIMICA ET BIOPHYSICA ACTA vol. 1519, 2001, pages 78 - 84, XP004245535
- TOPCZEWSKI J. ET AL.: 'Cloning and characterization of aspergillus nidulans cys B gene encoding systeine synthase' CURRENT GENETICS vol. 31, no. 4, 1997, pages 348 - 356, XP002109934
- DATABASE DDBJ [Online] June 1999 NATORFF R. ET AL.: 'The aspergillus nidulans METR sulphur regulator belongs to bZUP transcriptional factors', XP002958970 Database accession no. (AF148535)
- MARZLUF GEORGE A.: 'Molecular genetics of sulfur assimilation in filamentous fungi and yeast' ANNUAL REVIEW OF MICROBIOLOGY vol. 51, 1997, pages 73 - 96, XP002958971
- PIOTROWSKA M. ET AL.: 'sconC, a gene involved in the regulation of sulphur metabolism in aspergillus nidulans, belongs to the SPK1 gene family' MOLECULAR AND GENERAL GENETICS vol. 264, 2000, pages 276 - 282, XP002958972

## Description

### Technical Field

The present invention relates to a transcription factor capable of regulating the expression of a sulfur-assimilatory gene, a gene encoding such transcription factor, a recombinant vector comprising such transcription factor gene, koji mold in which the expression of a sulfur-assimilatory gene transformed by such vector has been derepressed, and use of such koji mold.

### Background Art

Organisms are capable of regulating gene expression in response to environmental factors such as nutrients or other various factors. Gene expression is regulated at various phases, for example, initiation of transcription, continuation of transcription, mRNA stability, initiation of translation, and continuation of translation.

Sulfur is a component of a sulfur-containing amino acid and is also an important nutrient for organisms. A great deal of research has been undertaken concerning the regulation of genes involved with sulfur assimilation of eukaryotic microorganisms (sulfur-assimilatory genes). Many facts concerning repressors and activators have been elucidated particularly from research based on, for example, the analysis of mutants and the gene isolation for *Neurospora crassa* (Review; Marzluf et al., Anuu. Rev. Microbiol. 51: 73-96, 1997).

A transcription activator Cys3 and transcription repressors Scon1 and Scon2 are known as transcription factors involved with sulfur assimilation of *Neurospora crassa*. Cys3 binds to the upstream region of the cys-3 gene or scon-2 gene and activates the expression thereof. Also, Cys3 can bind to the upstream region of genes involved with sulfur uptake or assimilation, such as ars gene or cys-14 gene, to activate the expression thereof.

A great deal of research has also been undertaken concerning the regulation of genes involved with sulfur assimilation of *Aspergillus nidulans*. In spite of a great deal of effort, in *Aspergillus nidulans*, no transcription factor equivalent to Cys3 of *Neurospora crassa* had been identified. In 1999, however, a transcription factor MetR was reported. A metR mutant was able to employ only methionine as a sulfur source. MetR was found to be highly homologous to Cys3 in the DNA-binding site, although it was not substantially homologous thereto in other areas. The cys-3 gene was not able to complement the phenotype of the metR mutant (Naorff et al., Fungal Genet. Newsl. 46 (Suppl), 59, 1999). Further, the existence of four types of transcription repressors was suggested (Natorff et al., Molec. Gen. Genet. 238: 185-192, 1993). Among the aforementioned, SconB and SconC genes have been isolated.

Koji molds, for example, yellow-green koji molds, such as *Aspergillus oryzae, Aspergillus sojae*, and *Aspergillus tamarii*; black koji molds, such as *Aspergillus niger, Aspergillus awamori, Aspergillus usamii*, and *Aspergillus saitoi*; and white koji molds, such as *Aspergillus kawachii*, have been utilized in fermentation, brewing, or enzyme production for a long period of time. They are very useful as highly safe microorganisms. However, the regulation of genes involved with sulfur assimilation of such microorganisms has not been substantially elucidated. These useful koji molds are different from the aforementioned *Aspergillus nidulans* (i.e., which is the imperfect stage of *Emericella nidulans* which belongs to the genus *Emericella* (*Ascomycetes*) having the sexual stage), for example, in that their sexual stages are not identified (USP No. 6,090,607). Thus, findings concerning *Aspergillus nidulans* cannot be always applied thereto.

Yellow-green koji molds *Aspergillus oryzae* and *Aspergillus sojae* secrete a number of hydrolase including a proteolytic enzyme. Thus, they have been used in the production of seasonings such as soy sauce or soybean paste via proteolysis. Production of extracellular protease is known to be induced or derepressed due to a deficiency of any of carbon, nitrogen and sulfur sources in *Aspergillus oryzae*. In *Aspergillus oryzae*, the genetic mechanism for protease expression caused by the deficiency of carbon and nitrogen sources has been reported, while a mechanism of protease expression caused by the deficiency of sulfur sources has not yet been elucidated. In the case of *Aspergillus nidulans*, several analyses have been made concerning protease expression caused by the deficiency of sulfur sources, but the mechanism thereof has not yet been reported. Involvement of sulfur sources with the regulation of expression of extracellular exopeptidase such as aminopeptidase or carboxypeptidase in a yellow-green koji mold is not known.

### Disclosure of the Invention

An object of the present invention is to provide a transcription factor capable of regulating the expression of a sulfur-assimilatory gene of koji mold during culture and a gene encoding the same. It is another object of the present invention to provide an effective method for producing a proteolytic enzyme using koji mold. A further object of the present invention is to provide an effective method for producing foods by utilizing the aforementioned method for producing a proteolytic enzyme.

The present inventors have conducted concentrated studies in order to attain the above objects. As a result, they have succeeded in cloning transcription factor genes capable of regulating the expression of a sulfur-assimilatory gene from a yellow-green koji mold. This has led to the completion of the present invention.

Specifically, the present invention is as follows.
1) A protein of the following (a) or (b):
   (a) a protein, which comprises the amino acid sequence as shown in SEQ ID NO: 3; or
   (b) a protein, which comprises an amino acid sequence derived from the amino acid sequence as shown in SEQ ID NO: 3 by deletion, substitution, or addition of one to 50 amino acid residues, and which has functions of a transcription factor.
2) A protein, which comprises an amino acid sequence having 70% or higher sequence homology to the amino acid sequence as shown in SEQ ID NO: 3 or a fragment thereof, and which has functions of a transcription factor.
3) A transcription factor gene, which encodes the following protein (a) or (b):
   (a) a protein, which comprises the amino acid sequence as shown in SEQ ID NO: 3; or
   (b) a protein, which comprises an amino acid sequence derived from the amino acid sequence as shown in SEQ ID NO: 3 by deletion, substitution, or addition of one or from 2 to 50 amino acid residues, and which has functions of a transcription factor.
4) A transcription factor gene, which comprises DNA, which comprises the nucleotide sequence as shown in SEQ ID NO: 2.
5) A gene, which encodes the protein according to 2) above.
6) A transcription factor gene, which comprises DNA, which comprises a nucleic acid encoding the amino acid sequence as shown in SEQ ID NO: 3.
7) A transcription factor gene, which comprises DNA having 80% or higher sequence homology to DNA comprising the nucleotide sequence as shown in SEQ ID NO: 2, and which encodes a protein having functions of a transcription factor.
8) A recombinant vector, which comprises the gene according to 3), 4), 5), 6) or 7) above.
9) A koji mold, which comprises the recombinant vector according to 8) above.
10) A koji mold, wherein the gene according to 3), 4), 5), 6) or 7) above is expressed in the presence of a low-molecular-weight sulfur-containing substance.
11) The koji mold according to 9) or 10) above, which is capable of producing extracellular protease in the presence of a low-molecular-weight sulfur-containing substance.
12) The koji mold according to 9) or 10) above, which is capable of producing extracellular protease and extracellular exopeptidase in the presence of a low-molecular-weight sulfur-containing substance.
13) A koji mold, wherein the expression of a sulfur-assimilatory gene is derepressed.
14) The koji mold according to 9), 10), 11) or 12) above, wherein the expression of a sulfur-assimilatory gene is derepressed.
15) A method for producing protease and/or exopeptidase, which comprises culturing the koji mold according to any one of 9) to 14) above in a medium and optionally collecting protease and/or exopeptidase from the resulting culture.
16) A method for degrading a protein-containing substance, which comprises degrading the protein-containing substance with the koji mold according to any one of 9) to 14) above.
17) A method for producing a degradation product of a protein-containing substance, which comprises culturing the koji mold according to any one of 9) to 14) above, degrading a protein-containing substance with the resulting culture, and optionally collecting a degradation product of the protein-containing substance therefrom.
18) The method according to 15) above, wherein the medium comprises 0.5 mM or more low-molecular-weight sulfur-containing substance.

Hereinafter, the present invention is described in detail.

In the context of the present invention, in genus *Aspergillus* microorganisms (koji molds) that are used in fermentation, brewing, enzyme production and the like, a transcription regulatory factor (a transcription factor) for a sulfur-assimilatory gene of and a gene encoding the same were identified, functions thereof were clarified, and the involvement of such transcription factor with the ability to produce extracellular protease or extracellular exopeptidase was elucidated. Thus, according to the present invention, a transcription factor that is capable of derepressing the expression of a sulfur-assimilatory gene in the presence of a low-molecular-weight sulfur-containing substance and thereby enhancing the capacity of a host koji mold to produce extracellular protease or extracellular exopeptidase, and a gene encoding the same, was successfully obtained. Use of the transcription factor of the present invention and/or a gene encoding the same can provide favorable fermentation, brewing, enzyme production and the like using koji mold.

In the present invention, the term "koji mold" refers to koji mold that is used for food manufacturing. Kojimold of the present invention is yellow-green koji molds, such as *Aspergillus oryzae, Aspergillus sojae* and *Aspergillus tamarii*; black koji molds, such as *Aspergillus niger, Aspergillus awamori, Aspergillus usamii* and *Aspergillus saitoi*; and white koji molds, such as *Aspergillus kawachii*. The koji mold that is employed in the present invention is preferably a yellow-green koji mold, such as *Aspergillus oryzae, Aspergillus sojae* or *Aspergillus tamarii*, more preferably a yellow-green koji mold, such as *Aspergillus oryzae* or *Aspergillus sojae*, and most preferably *Aspergillus oryzae*.

### 1. The transcription factor and the transcription factor gene of the present invention

The term "transcription factor of the present invention" refers to a transcription factor protein that is capable of regulating the expression of a sulfur-assimilatory gene. In the present description of the application, the term "sulfur-assimilatory gene" refers to a gene that is involved in sulfur assimilation. More specifically, such gene encodes a protein that is involved in uptake of a low-molecular-weight sulfur-containing substance from a medium into a cell, degradation thereof, or supply of sulfur to the sulfur-containing amino acid synthesis pathway. Examples thereof include the arylsulfatase gene, the cholinesulfatase gene, the sulfate permease gene, and the sulfate reductase gene. The sulfur-assimilatory gene that is employed in the present invention is preferably the arylsulfatase or cholinesulfatase gene, and the arylsulfatase gene is particularly preferable. In the present description, the term "capable of regulating the expression of a sulfur-assimilatory gene" refers to the ability to alter the pattern of expression of a sulfur-assimilatory gene by functioning as a transcription factor. Specific examples of such ability include enhancement of gene expression, derepression of such expression, lowering of such expression, repression of such expression, and alteration of the timing of such expression. Whether or not the transcription factor of the present invention "is capable of regulating the expression of a sulfur-assimilatory gene" can be determined by, for example, observing the way that the pattern of expression of a specific sulfur-assimilatory gene changes in the presence of the transcription factor of the present invention from that in the absence of the transcription factor of the present invention. More specifically, such changes in the pattern of expression of a sulfur-assimilatory gene can be observed, for example, when assaying changes in the amount or activity of mRNA of the aforementioned gene or of a protein encoded by the gene or changes in the timing of the expression in accordance with a conventional technique. An example of the subject to be analyzed is enzyme activity of arylsulfatase encoded by the arylsulfatase gene.

The transcription factor of the present invention provides a host koji mold with the ability to produce protease or exopeptidase when functioning as a transcription factor. This protease or exopeptidase is preferably extracellular protease or extracellular exopeptidase. In the present description, the terms "functioning as a transcription factor" and "having functions of a transcription factor" indicate that a protein is capable of binding to DNA having a specific nucleotide sequence and regulating the expression of a corresponding gene. Specifically, whether or not the protein of the present invention "has functions of a transcription factor" can be verified in the following manner. For example, it can be verified by proving that the protein of the present invention can bind to DNA comprising the nucleotide sequence of SEQ ID NO: 25 located upstream of the sconB gene, that is deduced to bind to the MetR protein based on research concerning the binding of the Cys3 protein to the sequence located upstream of the scon-2 gene in *Neurospora crassa* (Proc. Natl. Acad. Sci., U.S.A., 92 (8): 3343-3347, 1995), or the nucleotide sequence of SEQ ID NO: 26 complementary thereto. Binding of the protein to the specific DNA can be proved by, for example, gel shift analysis.

The transcription factor of the present invention is a protein comprising the amino acid sequence as shown in SEQ ID NO: 3. However, the transcription factor of the present invention is not limited to the aforementioned protein as long as it has the functions of a transcription factor as mentioned above. The transcription factor of the present invention may be a protein comprising an amino acid sequence derived from the amino acid sequence as shown in SEQ ID NO: 3 by deletion, substitution, or addition of one or 2 to 50, preferably 2 to 10, (further preferably a few) amino acid residues and having functions of a transcription factor. Further, the transcription factor of the present invention may be a protein comprising an amino acid sequence having 70% or higher sequence homology to the amino acid sequence as shown in SEQ ID NO: 3 or a fragment thereof and having functions of a transcription factor. Furthermore, the transcription factor of the present invention may be a protein, which is capable of regulating the expression of a sulfur-assimilatory gene.

The term "the transcription factor gene of the present invention" refers to a gene encoding the aforementioned transcription factor protein of the present invention. The transcription factor gene of the present invention comprises DNA comprising the nucleotide sequence as shown in SEQ ID NO: 2. However, the transcription factor gene of the present invention is not limited to the aforementioned gene as long as it encodes the aforementioned transcription factor protein of the present invention. The transcription factor gene of the present invention can encode a protein comprising the amino acid sequence as shown in SEQ ID NO: 3 or a protein comprising an amino acid sequence derived from the amino acid sequence as shown in SEQ ID NO: 3 by deletion, substitution, or addition of one or 2 to 50, preferably 2 to 10, (and further preferably a few) amino acid residues and having functions of a transcription factor. Further, the transcription factor gene of the present invention can comprise DNA comprising the nucleotide sequence as shown in SEQ ID NO: 2 or DNA hybridizing under stringent conditions with DNA comprising a nucleotide sequence complementary with the DNA comprising the nucleotide sequence as shown in SEQ ID NO: 2 and encoding a protein having functions of a transcription factor. Furthermore, the transcription factor gene of the present invention may encode a protein comprising an amino acid sequence having 70% or higher sequence homology to the amino acid sequence as shown in SEQ ID NO: 3 or a fragment thereof and having functions of a transcription factor. Still further, the transcription factor gene of the present invention may comprise DNA comprising a nucleic acid encoding the amino acid sequence as shown in SEQ ID NO: 3.

The transcription factor gene of the present invention can be expressed via a gene expression technique known to those skilled in the art to produce the transcription factor of the present invention.

### 2. Obtainment of the transcription factor gene of the present invention

The transcription factor gene of the present invention is represented by SEQ ID NO: 1 and SEQ ID NO: 2. The transcription factor gene of the present invention can be prepared, for example (but are not limited to), by isolation from koji mold. For example, the transcription factor gene of the present invention can be isolated from koji mold in the following manner. At the outset, genomic DNA or cDNA prepared from koji mold in accordance with a conventional technique is employed as a template to amplify a portion of the transcription factor gene by PCR. The nucleotide sequence of the amplification product is then determined, and subsequently the surrounding nucleotide sequences are determined with primer walking method. Thus, a full-length nucleotide sequence of the transcription factor gene is obtained. Based on this nucleotide sequence, a primer is then designed at a position where a full-length transcription factor gene can be amplified. PCR is carried out using the aforementioned primer and using the genomic DNA or cDNA prepared from koji mold in accordance with a conventional technique as a template, and the amplification product is recovered and purified, which results in preparation of the transcription factor gene of the present invention. The portion amplified by the initial PCR is sandwiched by a pair of the PCR primers that had been designed at adequate positions in the gene. These PCR primers are preferably designed in the region that has been identified as being highly conserved in the transcription factor gene of the present invention. Such region can be determined by comparing the nucleotide sequences of the transcription factor genes, such as the sequences of SEQ ID NOs: 1 and 2, other nucleotide sequences of the transcription factor gene of the present invention, and nucleotide sequences of other transcription factor genes. The highly conserved region can be easily identified in accordance with a technique known to those skilled in the art, including more specifically, for example, use of multiple alignment programs (e.g., CLASTAL V and CLASTAL W; for example, CLASTAL W may also be available from websites on the Internet including the homepage of the National Institute of Genetics, Japan (http://www.ddbj.nig.ac.jp)). Once the genomic clone is obtained from the organism from which the gene of interest is to be obtained, the coding region of the gene on the genome can be identified by subsequently utilizing a cDNA clone obtained by RT-PCR.

An example of another method for isolating the transcription factor gene of the present invention is as follows. Labeled DNA or RNA comprising the nucleotide sequence as shown in SEQ ID NO: 1 or 2 or a sequence complementary thereto is employed as a probe, and a clone that hybridizes therewith under stringent conditions is selected from the genomic DNA library or cDNA library of the aforementioned koji mold prepared in accordance with a conventional technique. In such a case, a probe can be comprised of the full length or a part of the aforementioned sequence. Further, a fragment prepared by amplifying a highly conserved sequence by PCR using the genomic DNA or cDNA of the organism as a template can be used as a probe. The stringent conditions mean conditions that allow a specific hybrid signal to be distinguished from a non-specific hybrid signal, although the conditions vary depending on the hybridization system and the type, sequence, and length of probe to be employed. The stringent conditions can be established through alteration of hybridization temperature, washing temperature, or salt concentration. For example, if a non-specific hybrid is disadvantageously detected as an intense signal, a hybridization specificity can be raised by increasing hybridization and washing temperatures and optionally lowering salt concentration during washing step. If even specific hybrid signals cannot be detected, hybrids can be stabilized by lowering hybridization and washing temperatures and optionally increasing salt concentration during washing step. Such optimization can be easily carried out by researchers in this technical field.

A specific example of stringent conditions is as follows. A DNA probe is used, and hybridization is carried out overnight (for approximately 8 to 16 hours) with a 5x SSC, 1.0% (W/V) blocking solution for nucleic acid hybridization (Boehringer Mannheim), 0.1% (W/V) N-lauroyl sarcosine, and 0.02% (W/V) SDS. Washing is carried out two times using 0.1 to 0.5x SSC, 0.1% (W/V) SDS, preferably 0.1x SSC and 0.1% (W/V) SDS for 15 minutes each. The hybridization temperature and washing temperature are 55°C or higher, preferably 60°C or higher, more preferably 65°C or higher, and most preferably 68°C or higher.

DNA included in the scope of the transcription factor gene of the present invention, such as DNA encoding a protein comprising an amino acid sequence having 70% or higher, more preferably 80% or higher, and most preferably 90% or higher sequence homology to the amino acid sequence as shown in SEQ ID NO: 3 or a fragment thereof and having functions of the transcription factor of the present invention; and DNA exhibiting 80% or higher, and most preferably 90% or higher sequence homology to DNA of the nucleotide sequence as shown in SEQ ID NO: 2 and encoding a protein having functions of the transcription factor, can be identified based on hybridization as mentioned above. Alternatively, such DNA can be easily found, for example, via similarity search using the BLAST software, from among a group of DNAs with unknown functions that have been obtained by genome sequence analysis or other means or nucleotide sequence information accumulated in public databases. DNAs identified based on hybridization or sequence analysis can be isolated as a DNA fragment comprising the transcription factor gene of the present invention, by excising it from a plasmid clone containing the aforementioned DNA with an adequate restriction enzyme, or amplifying a region containing the DNA by PCR. The nucleotide sequence of the thus isolated transcription factor gene of the present invention can be modified by a variety of conventional mutagenesis techniques (for example site-specific mutagenesis). The present invention includes the transcription factor gene of the present invention in which such modification has been introduced as long as it encodes a protein having functions of a transcription factor.

In the aforementioned sequence analysis, sequences are preprocessed so as to become optimal conditions for comparison in order to determine sequence homology between two amino acid sequences or between two nucleotide sequences. For example, a gap is introduced into one of the sequences to optimize the alignment with another sequence. Thereafter, amino acid residues or nucleotides at each site are compared. When the amino acid residue or nucleotide at a given site in the first sequence is identical to that at the corresponding site in the second sequence, these sequences are identical to each other at that site. Sequence homology between two sequences is represented as a percentage of the number of identical sites between sequences in relation to the number of total sites (total amino acid residues or nucleotides).

Based on the above principle, sequence homology between two amino acid sequences or between two nucleotide sequences is determined by the algorithm of Karlin and Altschul (Proc. Natl. Acad. Sci. U.S.A., 87: 2264-2268, 1990; and Proc. Natl. Acad. Sci. U.S.A., 90: 5873-5877, 1993). The BLAST Program that utilizes such algorithm was developed by Altschul et al. (J. Mol. Biol. 215: 403-410, 1990). Further, Gapped BLAST is a program that determines sequence homology having sensitivity higher than that of BLAST (Nucleic Acids Res. 25: 3389-3402, 1997). The aforementioned programs are mainly used for searching for a sequence that is highly homologous to a given sequence in the database. These programs usually employ default values for parameters. These programs are available on the website of, for example, the National Center for Biotechnology Information, U.S.A. on the Internet.

When a sequence having significant sequence homology cannot be found with the BLAST software, a homologous sequence can be searched for in the database using the higher sensitive FASTA software (W. R. Pearson and D. J. Lipman, Proc. Natl. Acad. Sci., 85: 2444-2448, 1988). The FASTA software can be used on the website of, for example, the GenomeNet. The default values for parameters can also be used in this case. For example, the nr-nt database is used and the ktup value is set to 6 when a nucleotide sequence is searched for.

Each of the aforementioned methods is mainly used for searching for a homologous sequence in the database. In the present invention, homology analysis using the Genetyx Mac Ver. 11.1 (Software Development Co., Ltd.) can be used for determining sequence homology for each sequence combination. This program employs a process based on the Lipman-Pearson method (Science, 227: 1435-1441, 1985) that is often employed because of its high performance and high sensitivity. When sequence homology between nucleotide sequences is examined, a protein-encoding region (CDS or ORF) is used, if possible. As parameters, the Unit Size to compare is set to 2, the Pick up Location is set to 5, and results are represented in terms of percentage. Sequence homology of the alignment exhibiting the highest value is employed as a result. When overlap of 30%, 50%, or 70% or higher is not observed between a query sequence and a sequence aligned therewith, these sequences are not always deduced to be functionally correlated each other, and thus the obtained value in the case is not employed as a value indicating sequence homology between these two sequences. For example, if there is a region comprising of only about several nucleotides or amino acid residues that are completely identical to each other in two sequences, it is highly likely to be accidental. When a region that is identical in two sequences constitutes only about several percent of the whole, it is difficult to consider that these sequences similarly function as a whole, even if the aforementioned region comprises a specific functional motif.

The description given below can demonstrate that a protein encoded by the thus obtained transcription factor gene of the present invention has functions of a transcription factor and functions of regulating the expression of a sulfur-assimilatory gene.

The transcription factor of the present invention can be produced by expressing the transcription factor gene of the present invention. Expression of the transcription factor gene of the present invention, and recovery and purification of the protein can be carried out by techniques known to those skilled in the art. In a general method for expressing the transcription factor gene of the present invention, a recombinant vector comprising the aforementioned gene incorporated therein is first prepared. The present invention also includes a recombinant vector comprising the transcription factor gene of the present invention incorporated therein that is prepared in the following manner.

### 3. Preparation of recombinant vector

The recombinant vector of the present invention can be obtained by ligating the transcription factor gene of the present invention into an adequate vector. Any vector can be used as long as it enables the transcription factor of the present invention to be produced in a host transformed therewith. For example, a plasmid, cosmid, phage, virus, chromosome-integrated vector, or artificial chromosome vector can be used.

The aforementioned vectors may comprise marker genes that enable the selection of transformed cells. Examples of marker genes include genes that complement auxotrophy of the host, such as URA3 and niaD, and drug resistance genes such as ampicillin, kanamycin, or oligomycin resistance gene. The recombinant vector preferably comprises a promoter that enables the expression of the gene of the present invention in a host cell or other regulatory sequences (for example, enhancer sequence, terminator sequence, and polyadenylation sequence). Specific examples of a promoter include GAL1 promoter, amyB promoter, and lac promoter. When the recombinant vector of the present invention is used for *in vitro* protein synthesis, its ability to produce the transcription factor of the present invention within the host is not necessary. In the case, the vector may comprise transcription and translation initiation sites that are suitable for the *in vitro* protein synthesis system to be used. An example of such vector is pIVEX2.3-MCS (Roche Diagnostics).

### 4. In vitro protein synthesis

The transcription factor of the present invention can be synthesized *in vitro,* for example, by a conventional *in vitro* protein synthesis technique, using a recombinant vector prepared by integrating the transcription factor gene of the present invention into the vector used for *in vitro* protein synthesis as mentioned above. Such *in vitro* synthesis can be carried out using, for example, the Rapid Translation System RTS 100 *E. coli* HY Kit (Roche Diagnostics) in accordance with the instructions of the kit.

### 5. Gel shift analysis

The transcription factor of the present invention can be verified to be a protein having functions of a transcription factor via, for example, gel shift analysis. Such gel shift analysis can be carried out in accordance with a conventional procedure using the purified transcription factor of the present invention or the transcription factor of the present invention that was synthesized *in vitro.* Probe DNA may be a labeled double-stranded DNA comprising a nucleotide sequence to which the transcription factor of the present invention binds and having a length suitable for the method to be employed. For example, annealed synthetic DNA, PCR-amplified DNA, DNA excised from a plasmid and the like can be employed. Examples of a sequence to which the transcription factor of the present invention binds include the nucleotide sequence as shown in SEQ ID NO: 25 located upstream of the sconB gene that is known to bind to the MetR protein in *Neurospora crassa* and the nucleotide sequence as shown in SEQ ID NO: 26, which is complementary to the former sequence.

In gel shift analysis, for example, DNA having a nucleotide sequence to which the transcription factor of the present invention binds is labeled at its 5'-terminus with fluorescence, heat-denatured, and then gradually cooled for annealing. The annealed product is added to a binding reaction solution together with the transcription factor of the present invention that was synthesized *in vitro,* the mixture is allowed to react at room temperature for 20 minutes. The reaction product is electrophoresed on 8% polyacrylamide gel, and a band is observed using a detector for detecting fluorescence, such as the Model 4200L Sequencer (LI-COR). If a shifted band that has shifted in a sequence-specific manner in the presence of the transcription factor of the present invention is observed, the transcription factor of the present invention is found to specifically bind to DNA of the sequence.

### 6. Obtainment of transformant

The transformant of the present invention can be obtained by transforming a host with a recombinant vector comprising the transcription factor gene of the present invention incorporated therein. The host to be used may be a koji mold. The koji mold includes yellow-green koji molds *Aspergillus oryzae, Aspergillus sojae*, and *Aspergillus tamarii*; black koji molds *Aspergillus niger, Aspergillus awamori*, *Aspergillus usamii*, and *Aspergillus saitoi*; and white koji molds, such as *Aspergillus kawachii*. The aforementioned koji mold employed in the present invention is preferably a yellow-green koji mold, such as *Aspergillus oryzae, Aspergillus sojae*, or *Aspergillus tamarii*, more preferably a yellow-green koji mold, such as *Aspergillus oryzae* or *Aspergillus sojae*, and most preferably *Aspergillus oryzae*.

Transformation can be carried out by a known method for transforming a koji mold. For example, it can be carried out by a method comprising forming a protoplast and then using polyethylene glycol and calcium chloride (Mol. Gen. Genet., 218: 99-104, 1989).

In the thus obtained koji mold, expression of the transcription factor gene of the present invention can be enhanced with the use of a recombinant vector that can express the aforementioned gene in a koji mold. In such a case, a recombinant vector that can enhance the expression of the transcription factor gene of the present invention in a koji mold can comprise a sequence element such as a promoter that is capable of inducing expression of gene in the recombinant vector.

A koji mold in which expression of the transcription factor gene of the present invention is enhanced can be also prepared by a method that is different from the aforementioned transformation technique utilizing a recombinant vector comprising the transcription factor gene of the present invention incorporated therein. Specifically, the koji mold in which expression of the transcription factor gene of the present invention is enhanced can be obtained by, for example, ultraviolet irradiation or radiation to koji mold or treating koji mold with mutagens such as nitrosoguanidine or ethylmethane sulfonate.

The present invention includes a transformant obtained with the use of a recombinant vector comprising the transcription factor gene of the present invention incorporated therein as mentioned above and a koji mold in which expression of the transcription factor gene of the present invention is enhanced.

### 7. Derepression of sulfur-metabolizing gene expression

In the koji mold obtained in the section 6 above, the pattern of the sulfur-assimilatory gene expression is changed. In a specific embodiment, repression of the expression of the sulfur-assimilatory gene by a low-molecular-weight sulfur-containing substance is lifted by the transcription factor of the present invention in the presence of a low-molecular-weight sulfur-containing substance in the koji mold of the present invention. This can be verified in a following manner.

Koji mold strains to be tested and the wild-type strains (host strains) are independently precultured in media containing low-molecular-weight sulfur-containing substances. Cultures are transferred to a medium that contains a low-molecular-weight sulfur-containing substance and a medium that does not contain it, and further cultured. Any type of low-molecular-weight sulfur-containing substances may be used as long as they can repress the expression of a sulfur-assimilatory gene in a wild-type strain. Examples thereof that can be used include methionine and sulfate. The concentration of a low-molecular-weight sulfur-containing substance is, for example, 0.5 mM or higher, preferably 1 mM or higher, more preferably 5 mM or higher, and most preferably 10 mM or higher. Cells are recovered from these cultures, and then lysed. Cell lysis can be carried out by, for example, placing cells in a mortar filled with liquid nitrogen, grinding the cells with a pestle, transferring it in a microtube with a buffer and glass beads, and lysing the cells using a Multi-beads shocker MB-200 (Yasui Kikai Corporation). Subsequently, the enzyme activity of a sulfur-assimilatory gene product in the lysate or that in a supernatant thereof prepared by centrifugation is measured. When the activity in the tested strains is at least 1.5 times, preferably at least 2 times, more preferably at least 3 times, still more preferably at least 5 times, and most preferably at least 10 times higher than that in wild-type strains in the presence of a low-molecular-weight sulfur-containing substance, it can be said that the repression of the sulfur-assimilatory gene expression by a low-molecular-weight sulfur-containing substance is lifted. When the enzyme activity of a sulfur-assimilatory gene product for culturing in the presence of a low-molecular-weight sulfur-containing substance is at least 10%, preferably 20%, and most preferably 30% or higher than that for culturing in the absence thereof, the repression can be said to be lifted. Under some conditions, some types of enzymes are known to appear to be temporarily derepressed in a wild-type strain during the process of growth (Biochem. J., 166: 415-420, 1977), and the results measured under such conditions are not employed. In order to determine such conditions, it is required to verify enzyme activity in a control experiment using a wild-type strain. An enzyme activity of the sulfur-assimilatory gene product to be measured includes the arylsulfatase activity. The arylsulfatase activity can be measured by a method in which p-nitrophenol sulfate is employed as a substrate (Biochem. J., 166: 411-413, 1977).

### 8. A koji mold having capacity for producing extracellular protease and extracellular exopeptidase higher than that of parent strain

The koji mold of the present invention is capable of producing extracellular protease and extracellular exopeptidase in the presence of a low-molecular weight sulfur-containing substance. In particular, the koji mold in which the expression of a sulfur-assimilatory gene in the presence of a low-molecular-weight sulfur-containing substance is derepressed by the transcription factor of the present invention has a capacity for producing extracellular protease and extracellular exopeptidase higher than that of the parent strain used in introducing the aforementioned genetic modification to a host. This was elucidated by the present invention. Accordingly, a koji mold having a capacity for producing extracellular protease and extracellular exopeptidase higher than that of the parent strain can be obtained by preparing the koji mold of the present invention, for example, in the aforementioned manner. The capacity for producing these enzymes can be tested by, for example, culturing the koji mold in accordance with a method for producing enzymes as described below, and measuring protease and exopeptidase activities in a medium by a conventional technique. Protease activity can be measured by, for example, a method using azocasein as a substrate (the Journal of the Soysauce Information Center, 16: 86-89, 1990). Activity of exopeptidase such as aminopeptidase can be measured by, for example, the method disclosed in JP Patent Publication (Kokai) No. 11-346777 A (1999), in which leucine-p-nitroanilide or leucyl-glycylglycine is employed as a substrate.

### 9. Method for producing enzymes of the present invention

A method for producing enzymes of the present invention comprises culturing the koji mold of the present invention in a medium and optionally recovering enzymes (e.g., protease or exopeptidase) from the culture. Culturing may be carried out via a liquid or solid culture technique. A medium to be used may comprise a low-molecular-weight sulfur-containing substance (i.e., a sulfur-containing substance that has a low molecular weight or that can be easily degraded to small molecules). In such medium, expression of a sulfur-assimilatory gene is repressed in wild-type koji molds. In contrast, such gene expression is derepressed in the koji mold of the present invention, and the capacity for producing extracellular protease or extracellular exopeptidase can be enhanced. This effect is particularly significant when the concentration of a low-molecular-weight sulfur-containing substance in a medium is at least 0.5 mM, preferably at least 1 mM, more preferably at least 2 mM, further preferably at least 3 mM, still more preferably at least 5 mM, and most preferably at least 10 mM. Depending on the type of a promoter used for regulating the expression of the transcription factor gene of the present invention, an inducible substrate for the promoter is preferably present in a medium according to need, and the concentration of the repressor for it in a medium is preferably low, concerning the koji mold in which the expression of the transcription factor of the present invention is enhanced by the recombinant vector of the present invention. An example of a promoter that can be used is an amylase gene promoter. In such a case, when the amount of the inducible substrate such as starch, dextrin, or maltose is considered to be small, any inducible substance may be added to accelerate the expression of the transcription factor of the present invention. In this case, the glucose concentration is preferably low.

Any culture temperature and culture period may be employed as long as an enzyme of interest can be produced under such condition. The produced enzyme may be used while being contained in a culture depending on the intended use. Alternatively, the enzyme is optionally extracted from the culture in accordance with a conventional technique and then partially or fully purified before use.

### 10. Use of the koji mold of the present invention in degradation of protein-containing substances

The koji mold of the present invention can be used for degrading protein-containing substances. Specifically, the koji mold of the present invention has a high capacity for producing extracellular protease and extracellular exopeptidase. Thus, the use thereof enables the efficient degradation of protein-containing substances. In the present description, the term "protein-containing substance" refers to a substance that comprises a protein as a constituent thereof. Examples thereof include: plant protein-containing substances such as soybeans, defatted soybeans, wheat, and wheat gluten; animal protein-containing substances such as milk, skimmed milk, milk casein, animal meat, and gelatin; fish-derived protein-containing substances such as fish meat or fish protein; microorganism-derived protein-containing substances such as yeast and Chlorella; and processed products thereof. In the method for degrading protein-containing substances of the present invention, the koji mold of the present invention can be first cultured in accordance with the method for producing enzymes described in the section 9 above, and the produced enzyme can be mixed with a protein-containing substance to facilitate the degradation reaction. Alternatively, a protein-containing substance may be inoculated with the koji mold of the present invention to allow the koji mold to grow in the protein-containing substance, thereby producing enzymes therein. In such a case, the reaction can be further accelerated by adequate processing, such as mixing with saline after the growth of the koji mold. Such examples are equivalent to, for example, koji making and mashing steps during the process of producing soy sauce or soybean paste. In particular, in wild-type koji molds within the media that are used in the production of soy sauce or soybean paste, i.e., protein-containing substances, often contain abundant sulfur-containing substances that have low molecular weights or that can be easily degraded to small molecules, the aforementioned expression is repressed by a low-molecular-weight sulfur-containing substance. In contrast, the koji mold of the present invention in which expression of sulfur-assimilatory enzymes is derepressed in the presence of a low-molecular weight sulfur-containing substance is very useful since it can efficiently produce proteolytic enzymes such as protease in, for example, the aforementioned process for producing soy sauce or soybean paste involving degradation of protein-containing substances. The present invention also includes a method for degrading protein-containing substances using the koji mold of the present invention. According to the present method for degrading protein-containing substances using the koji mold of the present invention, the degradation product of protein-containing substances can first be prepared as a degradation mixture. The degradation product of protein-containing substances may be used in such mixture according to need. Alternatively, the degradation product of interest may be separated from the above-mentioned degradation mixture before use, if necessary.

### Best Modes for Carrying out the Invention

The present invention is hereafter described in greater detail with reference to the following examples, although the technical scope of the present invention is not limited to these examples.

### [Example 1]

### Obtainment of a transcription factor gene that regulates a sulfur-assimilatory gene of the yellow-green koji mold Aspergillus oryzae

Spores of the yellow-green koji mold *Aspergillus oryzae* RIB 40 strain (ATCC 42149) were inoculated into 100 ml of YPD medium and cultured overnight with shaking at 30°C. Thereafter, genomic DNA was extracted in accordance with the method of Iimura (Argric. Biol. Chem., 323-328, 51 (1987)). PCR was carried out using this genomic DNA fragment as a template and using the following primers that had been synthesized based on the nucleotide sequences of metR, which is a regulatory factor gene for a sulfur-assimilatory gene of *Aspergillus nidulans*, and of cys-3, which is a regulatory factor gene for a sulfur-assimilatory gene of *Neurospora crassa*.
5'-ACCGC(T/C)GC(T/C)AGCGC(T/C)CG(A/G)TT-3' (SEQ ID NO: 7)
5'-(G/C)GA(G/T)CC(A/G)TG(T/C)TTCTC(A/G)GT-3' (SEQ ID NO: 8)

PCR was carried out using an Expand-HF (Roche Diagnostics) in a DNA Thermal Cycler (Takara Shuzo Co., Ltd.). The composition of the reaction solution is as shown below.

### (Reagent/volume used/final concentration)

H₂O / 36 µl
10x Reaction buffer / 5 µl / 1x
dNTP Mix (2.5 mM) / 5 µl / 250 µM
Primers / 1 µl for each of 2 types / 20 µM
Template (0.5 µg of DNA) / 1 µl
Expand-HF DNA Polymerase Mix / 1 µl / 3.5 U per experiment
Total liquid volume: 50 µl

The ingredients of the above reaction solution (50 µl) were mixed in a 0.2 ml reaction tube, the tube was mounted on the DNA Thermal Cycler, and PCR was carried out under the following temperature conditions:
1 cycle of 94°C for 3 minutes;
30 cycles of 94°C for 1 minute, 50°C for 1 minute, and 72°C for 1 minute; and
1 cycle of 72°C for 7 minutes.

The amplified product was confirmed via 1.5% agarose gel electrophoresis, the DNA fragment of interest was isolated, purified, and then ligated to the pT7Blue T-Vector for TA cloning (Novagen). The *E. coli* JM 109 strain (ATCC 53323) was transformed with the resultant vector, and cloned. A plasmid was prepared from the *E*. *coli* clone having the DNA fragment of interest, the nucleotide sequence of the cloned DNA fragment was analyzed, and homology analysis was performed using the NCBI blastx (http://www.ncbi.nlm.nih.gov/BLAST/). As a result, this sequence was found to be homologous to the *Aspergillus nidulans* MetR.

A genomic DNA clone comprising the full-length gene was then obtained in accordance with the method of Chenchik et al. (Biotechniques, 21: 526-534, 1996).

Specifically, the extracted genomic DNA was completely digested with each of restriction enzymes, including *Eco*RV, *Sca*I, *Dra*I, *Pvu*II, and *Ssp*I, that recognize and cleave a 6-bp sequence to generate blunt-ends. Thereafter, adaptors of (P)5'-ACCTGCCC-3'(NH₂) (SEQ ID NO: 9) and 5'-CTAATACGACTCACTATAGGGCTCGAGCGGCCGCCCGGGCAGGT-3' (SEQ ID NO: 10) were ligated to the both ends of the genomic DNA fragment. PCR was carried out using this genomic DNA fragment as a template and using a primer synthesized based on the nucleotide sequence determined above and a primer 5'-CTAATACGACTCACTATAGGGC-3' (SEQ ID NO: 11) having a part of the adaptor sequence. The following two primers were used when the 5' region was obtained.
5'-TTTTCCATCTCCAGCTGGGCTACGCG-3' (SEQ ID NO: 12)
5'-AGGGATCTGCGTGCTGTACTTGGTGTGT-3' (SEQ ID NO: 13)

The following primer was used when then 3' region was obtained.
5'-TGGAACGGACAGTGCGAGAGACTA-3' (SEQ ID NO: 14)

PCR was carried out using the Expand-HF in the DNA Thermal Cycler. *Dra*I was used when the 5' region and the 3' region were obtained. The composition of the reaction solution is as shown below.

### (Reagent/amount used/final concentration)

H₂O / 18.25 µl
10x Reaction buffer / 2.5 µl / 1x
dNTP Mix (2.5 mM) / 2.5 µl / 250 µM
Primers / 0.25 µl for each of 2 types / 5 µM
Template (0.2 µg of DNA) / 1 µl
Expand-HF DNA Polymerase Mix / 0.25 µl / 1.25 U per experiment
Total liquid volume: 25 µl

The ingredients of the above reaction solution (25 µl) were mixed in a 0.2 ml reaction tube, the tube was mounted on the DNA Thermal Cycler, and step-down PCR was carried out under the following temperature conditions:
1 cycle of 95°C for 1 minute;
3 cycles of 95°C for 30 seconds, 74°C for 15 seconds, and 70°C for 3 minutes;
3 cycles of 95°C for 30 seconds, 70°C for 15 seconds, and 70°C for 3 minutes; 3
3 cycles of 95°C for 30 seconds, 66°C for 15 seconds, and 70°C for 3 minutes;
3 cycles of 95°C for 30 seconds, 62°C for 15 seconds, and 70°C for 3 minutes;
3 cycles of 95°C for 30 seconds, 58°C for 15 seconds, and 70°C for 3 minutes; and
20 cycles of 95°C for 30 seconds, 54°C for 15 seconds, and 70°C for 3 minutes.

The amplified product was confirmed via 1% agarose gel electrophoresis, the DNA fragment of interest was isolated, purified, and then ligated to the pT7Blue T-Vector for TA cloning. The *E*. *coli* JM 109 strain was transformed with the resultant vector, and cloned. A plasmid was prepared from the *E*. *coli* clone having the DNA fragment of interest, and the nucleotide sequence of the cloned DNA fragment was analyzed. This nucleotide sequence is shown in SEQ ID NO: 1. This sequence was subjected to homology search using the NCBI blastx (http://www.ncbi.nlm.nih.gov/BLAST/). As a result, it was found to be homologous to the *Aspergillus nidulans* MetR.

cDNA was amplified by RT-PCR in order to determine the nucleotide sequence of the cDNA of the aforementioned gene. Spores of the *Aspergillus oryzae* RIB 40 strain were inoculated into 100 ml of YPD medium and cultured at 30°C for 20 hours. Thereafter, cells were recovered, and then total RNAs were obtained in accordance with the method of Chigwin et al. (Biochemistry 18, 5294-8299, 1979). Then, mRNA was obtained using the oligo (dT) cellulose column (Amersham). Thereafter, cDNA was synthesized using the Ready-To-Go RT-PCR Beads (Amersham). The following two primers were used.
5'-ATGTCAGATGAGCACATCGCTCGTCAG-3' (SEQ ID NO: 15)
5'-CTAGTTATCGGTGCCCACACCCTTC-3' (SEQ ID NO: 16)

The composition of the reaction solution was determined in accordance with the standard composition of the kit. Reaction conditions were as follows:
42°C for 30 minutes;
95°C for 5 minutes; and
32 cycles of 95°C for 30 seconds, 55°C for 1 minute, and 72°C for 1 minute.

The amplified product was confirmed via 1% agarose gel electrophoresis, the DNA fragment of interest was isolated, purified and then ligated to the pT7Blue T-Vector for TA cloning (Novagen). The *E*. *coli* JM 109 strain was transformed with the resultant vector and cloned. A plasmid was prepared from the *E. coli* clone having the DNA fragment of interest, and the nucleotide sequence of the cloned DNA was analyzed. A portion that is present in the sequence of this gene on the chromosome determined above but is absent from this cloned fragment was determined to be an intron. The nucleotide sequence of this cDNA was analyzed to reveal the existence of the 831-bp open reading frame (hereafter abbreviated as "ORF"). The nucleotide sequence of this ORF is shown in SEQ ID NO: 2. The aforementioned protein gene of the chromosomal DNA and that of the cDNA, which were obtained in the above nucleotide sequencing, were compared each other. As a result, one intron of 732-bp was found to exist on the chromosomal DNA. The amino acid sequence deduced from the nucleotide sequence of cDNA is shown in SEQ ID NO: 3. The result of the analysis of this amino acid sequence indicates the existence of a sequence in the vicinity of the C-terminus of the protein that can have a leucine zipper structure. This indicates that the protein according to the present invention is a DNA-binding protein. A sequence having high sequence identity with this amino acid sequence was searched for from a conventional database for amino acid sequences. Search was conducted using the NCBI blastp (http://www.ncbi.nlm.nih.gov/BLAST/) and the assigned nr database. There was no matching sequence, and the MetR protein of *Aspergillus nidulans* (Accession AAD 38380) had the highest sequence homology. These sequences were subjected to homology search using the Genetyx Mac Ver. 11.1, and they were found to share 28.8% sequence homology in a 257-residue overlap. With this sequence homology of as low as 28.8%, therefore, it was difficult to presume that the protein encoded by the obtained gene had functions similar to those of MetR based on the sequence homology alone.

A sequence that was highly homologous to the nucleotide sequence as shown in SEQ ID NO: 2 was searched for. Search was conducted using the NCBI blastn (http://www.ncbi.nlm.nih.gov/BLAST/) and the assigned nr database. The cys-3 gene of *Neurospora crassa* was found to have the highest homology therewith, but the matching region was limited. Also, the metR gene of *Aspergillus nidulans* was not included in the search results. Thus, search was further conducted using FASTA, which has higher sensitivity. The FASTA search service of the GenomeNet (http://www.genome.ad.jp) was employed, and the nr-nt database was used. As a result, the metR gene (Accession AF 148535) of *Aspergillus nidulans* was found to have the highest sequence homology. The CDS region (ORF region) was extracted from this sequence and investigated by homology search with the Genetyx Mac Ver. 11.1, and the sequences were found to share 50% homology in a 793-nucleotide overlap.

Further, DNA encoding an amino acid sequence that was homologous to the amino acid sequence as shown in SEQ ID NO: 3 was searched for. Search was conducted using the NCBI tblastn (http://www.ncbi.nlm.nih.gov/BLAST/) and the assigned nr database. As a result, the metR gene of *Aspergillus nidulans* (Accession AF 148535) was found to have the highest sequence homology therewith, and sequence homology at the amino acid level was as described above.

### [Example 2]

### Determination of partial sequence of sconB gene of yellow-green koii mold

Spores of the filamentous fungi *Aspergillus oryzae* RIB 40 strain (ATCC 42149) were inoculated into 100 ml of YPD medium and cultured overnight with shaking at 30°C. Thereafter, genomic DNA was extracted in accordance with the aforementioned method of Iimura. PCR was carried out using this genomic DNA fragment as a template and using the following two primers, which had been synthesized based on the nucleotide sequences of the meaB gene of *Aspergillus nidulans* and that of *Neurospora crassa*.
5'-CG(G/A)ATGTG(T/C)GAACAACAC-3' (SEQ ID NO: 17)
5'-CAA(A/C)CCCTC(G/C)AGGTG(A/C)CCGAA-3' (SEQ ID NO: 18)

PCR was carried out using the Expand-HF in the DNA Thermal Cycler. The composition of the reaction solution is as shown below.

### (Reagent/volume used/final concentration)

H₂O / 36 µl
10x Reaction buffer / 5 µl / 1x
dNTP Mix (2.5 mM) / 5 µl / 250 µM
Primers / 1 µl for each of 2 types / 20 µM
Template (0.5 µg of DNA) / 1 µl
Expand-HF DNA Polymerase Mix / 1 µl / 3.5 U per experiment
Total liquid volume: 50 µl

The ingredients of the above reaction solution (50 µl) were mixed in a 0.2 ml reaction tube, the tube was mounted on the DNA Thermal Cycler, and PCR was carried out under the following temperature conditions:
1 cycle of 94°C for 3 minutes;
30 cycles of 94°C for 1 minute, 55°C for 1 minute, and 72°C for 1 minute; and
1 cycle of 72°C for 7 minutes.

The amplified product was confirmed via 1.0% agarose gel electrophoresis, the DNA fragment of interest was isolated, purified, and then ligated to the pT7Blue T-Vector for TA cloning. The *E*. *coli* JM 109 strain was transformed the resultant vector, and cloned. A plasmid was prepared from the *E. coli* clone having the DNA fragment of interest, the nucleotide sequence of the cloned DNA fragment was analyzed, and homology search was performed using the NCBI blastx (http://www.ncbi.nlm.nih.gov/BLAST/). As a result, this sequence was found to be highly homologous to the *Aspergillus nidulans* SconB.

A genomic DNA clone comprising the full-length gene was then obtained in accordance with the method of Chenchik et al. (Biotechniques, 21: 526-534, 1996).

Specifically, the extracted genomic DNA was completely digested with each of restriction enzymes, including *Eco*RV, *Sca*I, *Dra*I, *Pvu*II, and *Ssp*I, that recognize and cleave a 6-bp sequence to generate blunt-ends. Thereafter, adaptors of (P)5'-ACCTGCCC-3'(NH₂) (SEQ ID NO: 9) and 5'-CTAATACGACTCACTATAGGGCTCGAGCGGCCGCCCGGGCAGGT-3' (SEQ ID NO: 10) were ligated to the both ends of the genomic DNA fragment. PCR was carried out using this genomic DNA fragment as a template and using a primer synthesized based on the nucleotide sequence determined above and a primer 5'-CTAATACGACTCACTATAGGGC-3' (SEQ ID NO: 11) having a part of the adaptor sequence. The following primer was used when the 5' region was obtained.
5'-AGGAAGCCCCCAGCCACATTTCTTGC-3' (SEQ ID NO: 19)

The following primer was used when then 3' region was obtained.
5'-ACGATTCTTGCCTCAGCCTCCG-3' (SEQ ID NO: 20)

PCR was carried out using the Expand-HF in the DNA Thermal Cycler. *Dra*I was used when the 5' region and the 3' region were obtained. The composition of the reaction solution is as shown below.

### (Reagent/volume used/final concentration)

H₂O / 18.25 µl
10x Reaction buffer / 2.5 µl / 1x
dNTP Mix (2.5 mM) / 2.5 µl / 250 µM
Primers / 0.25 µl for each of 2 types / 5 µM
Template (0.2 µg of DNA) / 1 µl
Expand-HF DNA Polymerase Mix / 0.25 µl / 1.25 U per experiment
Total liquid volume: 25 µl

The ingredients of the above reaction solution (25 µl) were mixed in a 0.2 ml reaction tube, the tube was mounted on the DNA Thermal Cycler, and step-down PCR was carried out under the following temperature conditions:
1 cycle of 95°C for 1 minute;
3 cycles of 95°C for 30 seconds, 74°C for 15 seconds, and 70°C for 3 minutes;
3 cycles of 95°C for 30 seconds, 70°C for 15 seconds, and 70°C for 3 minutes;
3 cycles of 95°C for 30 seconds, 66°C for 15 seconds, and 70°C for 3 minutes;
3 cycles of 95°C for 30 seconds, 62°C for 15 seconds, and 70°C for 3 minutes;
3 cycles of 95°C for 30 seconds, 58°C for 15 seconds, and 70°C for 3 minutes; and
20 cycles of 95°C for 30 seconds, 54°C for 15 seconds, and 70°C for 3 minutes.

The amplified product was confirmed via 1% agarose gel electrophoresis, an approximately 1- to 2-kb DNA fragment was isolated, purified, and then ligated to the pT7Blue T-Vector for TA cloning. The *E. coli* JM 109 strain was transformed with the resultant vector, and cloned. A plasmid was prepared from the *E. coli* clone having the DNA fragment of interest, and the nucleotide sequence of the cloned DNA fragment was analyzed. This nucleotide sequence is shown in SEQ ID NO: 4. This sequence was subjected to homology search using the NCBI blastx (http://www.ncbi.nlm.nih.gov/BLAST/). As a result, it was found to be homologous to the *Aspergillus nidulans* SconB.

cDNA was amplified by RT-PCR in order to determine the nucleotide sequence of the cDNA of the aforementioned gene. Spores of the *Aspergillus oryzae* RIB 40 strain were inoculated into 100 ml of YPD medium and cultured at 30°C for 20 hours. Thereafter, cells were recovered, and then total RNAs were obtained in accordance with the method of Chigwin et al. Then, mRNA was obtained using the oligo (dT) cellulose column. Thereafter, cDNA was synthesized using the Ready-To-Go RT-PCR Beads. The following two primers were used.
5'-ATGGCCCAACCGACCGGAGAACTTAC-3'(SEQ ID NO: 21)
5'-TTAATTGCGGAAACTGTACATGCGCA-3'(SEQ ID NO: 22)

The composition of the reaction solution was determined in accordance with the standard composition of the kit. Reaction conditions were as follows:
42°C for 30 minutes;
95°C for 5 minutes; and
32 cycles of 95°C for 30 seconds, 55°C for 1 minute, and 72°C for 1 minute.

The amplified product was confirmed via 1% agarose gel electrophoresis, the DNA fragment of interest was isolated, purified, and then ligated to the pT7Blue T-Vector for TA cloning. The *E. coli* JM 109 strain was transformed with the resultant vector, and cloned. A plasmid was prepared from the *E*. *coli* clone having the DNA fragment of interest, and the nucleotide sequence of the cloned DNA fragment was analyzed. A portion that is present in the chromosomal DNA-derived nucleotide sequence determined above but is absent from this cloned fragment was determined to be an intron. The nucleotide sequence of this cDNA was analyzed. This analysis revealed the existence of the 2,055-bp open reading frame. The nucleotide sequence thereof is shown in SEQ ID NO: 5. The aforementioned protein gene of the chromosomal DNA and that of the cDNA, which were obtained in the above nucleotide sequencing, were compared each other. As a result, one intron of 48-bp was found to exist on the chromosomal DNA. The amino acid sequence deduced from the aforementioned nucleotide sequence is shown in SEQ ID NO: 6. A sequence having high sequence identity with this amino acid sequence was searched for from a conventional database for amino acid sequences. Search was conducted using the NCBI blastp (http:Hwww.ncbi.nlm.nih.gov/BLAST/) and the assigned nr database. There was no matching sequence, and the SconB protein of *Aspergillus nidulans* (Accession Q00659) had the highest sequence homology. These sequences were subjected to homology search using the Genetyx Mac Ver. 11.1, and the sequences were found to share 80.1% sequence homology in a 677-residue overlap. The protein encoded by the obtained gene was highly homologous to the *Aspergillus nidulans* SconB in an approximately 99% overlap relative to the whole; therefore, these sequences were considered to have substantially the same functions.

A sequence that was highly homologous to the nucleotide sequence as shown in SEQ ID NO: 4 was searched for. Search was conducted using the NCBI blastn (http://www.ncbi.nlm.nih.gov/BLAST/) and the assigned nr database. The SconB gene of *Aspergillus nidulans* (Accession U21220) was found to exhibit the highest homology. Thus, the CDS region (ORF region) was extracted from this sequence and investigated by homology search with the Genetyx Mac Ver. 11.1. As a result, the sequences were found to share 73.2% sequence homology in a 2,036-nucleotide overlap.

Further, DNA encoding an amino acid sequence that was homologous to the amino acid sequence as shown in SEQ ID NO: 6 was searched for. Search was conducted using the NCBI tblastn (http://www.ncbi.nlm.nih.gov/BLAST/) and the assigned nr database. As a result, the SconB gene of *Aspergillus nidulans* (Accession U21220) was found to exhibit the highest sequence homology, and sequence homology at the amino acid level was as described above.

### [Example 3]

### In vitro protein synthesis

PCR was carried out using the cDNA prepared in Example 1 as a template and using the following two primers.
5'-GAAGGAGATATACATATGGATTACGACCAG-3' (SEQ ID NO: 23)
5'-CCCCCGGGAGCTCCTAGTTATCGGTGCCCA-3' (SEQ ID NO: 24)

The amplified fragment was inserted into the *Nde*I-*Sac*I site of the expression vector pIVEX2.3-MCS (Roche Diagnostics). The reaction was allowed to proceed using the Rapid Translation System RTS 100 *E*. *coli* HY Kit (Roche Diagnostics) at 30°C for 4 hours. Thus, a protein having the amino acid sequence as shown in SEQ ID NO: 3 was expressed.

The reaction system is comprised of the following.
12 µl of *E. coli* lysate
10 µl of Reaction Mix
12 µl of amino acids
1 µl of methionine
5 µl of reconstruction buffer
1 µl of template DNA

These ingredients were mixed with each other, and the final volume of such mixture was adjusted to 50 µl with the addition of DNase/RNase-free distilled water.

The protein that was expressed in the *in vitro* protein synthesis system was subjected to the following gel shift analysis.

### [Example 4]

### Gel shift analysis

A leucine zipper structure was present on the C-terminal side of the amino acid sequence as shown in SEQ ID NO: 3. In order to verify that the protein according to the present invention has the functions of a transcription factor, specifically, protein functions as a DNA-binding protein, gel shift analysis was carried out using a promoter region of the sconB gene as a probe.

Double-stranded DNA was used as a probe for gel shift analysis. This double-stranded DNA was prepared by heating 5'-GGACGACTGCTACCACGGTAGCGCGCGGGC-3' (SEQ ID NO: 25) that has been labeled on its 5' end with IRDye-800 and unlabeled 5'-GCCCGCGCGCTACCGTGGTAGCAGTCGTCC-3' (SEQ ID NO: 26) at 94°C for 5 minutes, and gradually cooling them for annealing. In a binding reaction, 160 fmol of the probe prepared in the aforementioned reaction and 1 µl of the protein prepared in Example 3 were added to a binding reaction solution (10 mM Tris-HCl (pH 7.5), 50 mM NaCl, 0.5 mM DTT, 0.5 mM EDTA, 2 mM MgCl₂, 4% glycerol, and 0.2 mg/ml BSA) to be allowed to react at room temperature for 20 minutes, electrophoresis was carried out on 8% polyacrylamide gel (79: 1), and for detection, a shifted band was observed using the LI-COR 4200L Sequencer (LI-COR). As a result, a band shifted in a sequence-specific manner was observed with respect to the sequence. This strongly indicates that a protein having the amino acid sequence as shown in SEQ ID NO: 3 binds to a promoter region of the sconB gene and is involved with the regulation of the transcription thereof. The results of this experiment demonstrate that a protein having the amino acid sequence as shown in SEQ ID NO: 3 functions as a DNA-binding protein.

### [Example 5]

### Construction of a koii mold expression vector

A plasmid was prepared in which the argB gene (a marker gene) in the expression vector plasmid pMAR5 having an amylase promoter of *Aspergillus oryzae* (Biosci. Biotech. Biochem., 56: 1674-1675, 1992), was replaced with the pyrG gene of *Aspergillus oryzae*. pMAR5 was digested with *Sph*I, blunt-ended, further digested with *Sal*I, and electrophoresed on 0.7% agarose gel to obtain an approximately 4-kb fragment. Separately, ppyrG-26 (JP Patent Publication (Kokai) No. 2001-46053 A) was digested with *Bam*HI, blunt-ended, further digested with *Sal*I, and electrophoresed on 0.7% agarose gel to obtain an approximately 2.2-kb DNA fragment comprising the pyrG gene. These fragments were ligated to each other to generate a vector, and the *E*. *coli* JM 109 strain was transformed with it. The obtained plasmid referred to as pAP. This plasmid is an expression vector that comprises the pyrG gene as a selection marker and has the *Sma*I site between the promoter and the terminator of the amylase gene. This plasmid can express the gene of interest under control of the amylase gene promoter by incorporating the ORF of the gene in the same direction as the promoter into the *Sma*I site and transforming the koji mold with it.

RT-PCR was carried out using the RNA obtained in Example 1 as a template and using the RNA LA-PCR Kit (Takara Shuzo Co., Ltd.) to obtain a DNA fragment comprising the ORF having the nucleotide sequence as shown in SEQ ID NO: 2.

A primer attached to the kit, which comprises an adaptor sequence on the 3' side of oligo (dT), was used for reverse transcription. The composition of the reaction solution was determined in accordance with the standard composition of the kit.

Reverse transcription was carried out under temperature conditions consisting of 30°C for 10 minutes, 50°C for 30 minutes, 99°C for 5 minutes, and then 5°C for 5 minutes.

Subsequently, PCR was carried out by adding a primer, a thermostable polymerase, and other materials to the reverse transcription product in accordance with the instructions of the kit. The adaptor primer attached to the kit and the following primer were used, and amplification was initiated from a position immediately before the initiation codon on the 5' side and from the poly(A) site on the 3' side.
5'-CAAGCT TCCAATATGTCAGATGAGCA-3' (SEQ ID NO: 27)

PCR was carried out at 94°C for 2 minutes, 15 cycles of 94°C for 10 seconds, 53°C for 20 seconds, and 72°C for 1 minute and 20 seconds, followed by at 72°C for 5 minutes. A thermal cycler of DNA Engine PCT-200 (MJ Research) was employed, wherein temperatures were controlled by calculation control method.

Subsequently, PCR was carried out using the amplification product of the aforementioned RT-PCR as a template and using the above primer (SEQ ID NO: 28) and the following primer. Amplification was initiated from a position immediately before the initiation codon on the 5' side and from a position immediately following the termination codon on the 3' side.
5'-AAGCTCTAGATACACAAGGTAACAGA-3' (SEQ ID NO: 28)

Modification has been introduced into each primer, such that the amplified sequence comprises the recognition sequence of the restriction enzyme *Hind*III on its 5' side and the recognition sequence of the restriction enzyme *Xba*I on the 3' side. The Tbr EXT DNA polymerase (Fynnzymes) was used as a thermostable DNA polymerase, and the composition of the reaction solution was determined in accordance with the instructions attached to the polymerase.

PCR was carried out at 94°C for 2 minutes, 30 cycles of 94°C for 10 seconds, 53°C for 20 seconds, and 72°C for 1 minute, followed by at 72°C for 5 minutes. A portion of the amplification product was electrophoresed on 0.7% agarose gel, and an approximately 0.9-kb band was observed.

Subsequently, the amplification product was inserted into the pCR2.1-TOPO vector using the TOPO TA Cloning Kit with TOP 10 Cell (Invitrogen). The *E*. *coli* TOP 10 strain attached to the kit was transformed with it, and then 8 clones of transformants were obtained. A plasmid was extracted from each transformant, cleaved with restriction enzymes *Hind*III and *Xba*I, and electrophoresed on 0.7% agarose gel. As a result, fragments of approximately 0.9 kb were observed in all clones. The nucleotide sequences of these 8 clones were examined, and one clone free of PCR-introduced error was identified.

This plasmid was digested with *Hind*III and *Xba*I, blunt-ended, and electrophoresed on 0.7% agarose gel, and then an approximately 0.9-kb fragment was extracted. This fragment was ligated to *Sma*I-digested pAP to generate a vector, and the *E*. *coli* JM 109 strain was transformed with it. Plasmids were prepared from 6 clones of *E*. *coli* transformants, and the directions of the inserts were examined. As a result, 2 out of 6 clones were found to be have inserts in the suitable direction relative to the amylase promoter. One thereof was designated as pAM1. pAM1 was deposited at the National Institute of Advanced Industrial Science and Technology, the International Patent Organism Depositary, (Tsukuba Central 6, 1-1-1 Higashi, Tsukuba, Ibaraki, Japan) as of February 19, 2002, under the accession number: FERM BP-7907.

### [Example 6]

### Obtainment of koji mold transformant

The pyrG-deficient strains obtained from the *Aspergillus oryzae* 1764 strains (IFO4206, IAM2636) by the method disclosed in JP Patent Publication (Kokai) No. 2001-46053 A were transformed with pAM1. Transformation was carried out by a method involving protoplast formation and use of polyethylene glycol and calcium chloride (Mol. Gen. Genet., 218: 99-104, 1989). The pyrG-deficient strains were transformed using 5 µg of pAM1, transformants were selected in the minimal medium, and approximately 1,000 colonies were obtained. Among them, 12 colonies were repeatedly subjected to isolation of single conidia in the minimal medium to stabilize their traits. The conidia of these strains were scraped from the agar medium, suspended in 100 µl of 0.05% Triton X-100 in TE buffer, and then freezed in a deep freezer at -80°C and rethawed at room temperature three times. This procedure was repeated three times to obtain chromosomal DNA that can be used as a template for PCR. PCR was carried out using such chromosomal DNA as a template, and a primer of the sequence shown below, which corresponds to a sequence of the vector region located upstream of the amylase promoter and the primer having the sequence as shown in SEQ ID NO: 28 oriented upstream from the 3' terminus of the inserted gene.
5'-GAGCGGATAACAATTTCACACAGG-3' (SEQ ID NO: 29)

The Tbr EXT DNA polymerase (Fynnzymes) was used as a thermostable DNA polymerase, and the composition of the reaction solution was determined in accordance with the instructions attached to the polymerase.

PCR was carried out at 94°C for 2 minutes, followed by 45 cycles of 94°C for 10 seconds, 60°C for 20 seconds, and 72°C for 2 minutes. A portion of the amplification product was electrophoresed on 0.7% agarose gel, and approximately 2 kb bands were observed in 10 samples. Thus, these strains were found to have a region from the amylase promoter to the 3' terminus of the inserted gene without interruption. One strain thereof was designated as TFM1.

### [Example 7]

### Derepression of a koji mold sulfur-assimilatory gene

About 10⁶ cells of conidia of the TMF1 strain and of the *Aspergillus oryzae* 1764 parent strain (a wild-type phenotype for the regulatory system of the sulfur-assimilatory gene) were inoculated into 40 ml of peptone dextrin medium supplemented with 10 mM methionine, and rotary shaking culture was carried out at 150 rpm and 30°C for 24 hours, respectively. Cells were sedimented by centrifugation and the supernatant was discarded. Cells were suspended in 40 ml of ice-cold sterile ultrapure water and subjected to centrifugation again, for washing. After such washing had been repeated three times, cells were separately suspended in 50 ml of repressing medium (10 mM methionine in a sulfur-source-free minimal medium) and in 50 ml of derepressing medium (a sulfur source-free minimal medium). The resultants were transferred to Erlenmeyer flasks, and rotary shaking culture was carried out at 30°C and 150 rpm. Cells were separated by centrifugation 14 hours later and washed three times with 40 ml of ice-cold sterile ultrapure water in the same manner as described above. The washed cells were thoroughly drained using paper towels, transferred to a mortar filled with liquid nitrogen, and lysed with a pestle. About a half volume of the lysed cells was transferred to a microtube containing 0.7 ml of Tris-maleate buffer (0.2 M maleic acid-added 0.2 M Tris (pH 6.9)), 0.35 g of glass beads were added thereto, and cells were lysed using a Multi-beads shocker MB-200 (Yasui Kikai Corporation) at a power output of 80% for 10 minutes. Centrifugation was carried out at 10,000x g for 2 minutes to allow the glass beads and insoluble matter to become sedimented, and the supernatant of the lysate was recovered. The protein concentration of the lysate supernatant was measured using the protein assay kit II (Bio-Rad) and the supernatant was diluted to 0.4 mg/ml with the Tris-maleate buffer. 20 mM p-nitrophenol sulfate in Tris-maleate buffer (150 µl) was added to 25 µl of the lysate supernatant, and incubated at 37°C for 15 minutes. 0.5 M sodium hydroxide in aqueous solution (150 µl) was then added and agitated to terminate the reaction. In the blind test, the lysate was added after the addition of an aqueous sodium hydroxide, instead of before the addition thereof. The absorbance at 402 nm of each sample and that of the blind test subject were measured, and the difference in the absorbance between the sample and the blind test subject was determined to be the activity value of arylsulfatase (in arbitrary units). The results are shown in Table 1 below.

**Table 1**

| Strain | Medium | A420 | Scores for blind test subject | Difference in absorbance | Repression/derepression (%) |
|---|---|---|---|---|---|
| 1764 | Repressing medium | 0.324 | 0.306 | 0.018 | 2.5 |
| | Derepressing medium | 1.015 | 0.297 | 0.718 | |
| TFM1 | Repressing medium | 0.647 | 0.297 | 0.350 | 36.9 |
| | Derepressing medium | 1.251 | 0.303 | 0.948 | |

While the activity of the 1764 strain in the repressing medium was 2.5% of that in the derepressing medium, the activity of the TFM1 strain in the repressing medium was as high as 36.9% of that in the derepressing medium. The activity of the TFM1 strain was approximately 19 times higher than that of the parent strain in the repressing medium and approximately 1.3 times higher than that of the parent strain in the derepressing medium. This indicates that repression of sulfur-assimilatory gene expression by a low-molecular-weight sulfur-containing compound is lifted in this koji mold.

### [Example 8]

### Culturing of koji mold and assay of extracellular enzyme activity

The TFM1 strain and the *Aspergillus oryzae* 1764 strain were cultured in a variety of media, and extracellular enzyme activities were assayed.

### a. Solid culture on wheat bran medium

Water or 20 mM methionine in aqueous solution (2.22 ml) was added to 2.78 g of wheat bran, they were thoroughly mixed with each other, and the mixture was sterilized in a 150 ml-Erlenmeyer flask by autoclave at 120°C for 60 minutes. The aforementioned koji mold conidia (approximately 10⁶ cells) were inoculated therein, and standing culture was carried out at 30°C. The container was shaken 20 hours later to finely disperse the medium, and culture was continued for an additional 32 hours. After the completion of culture, 50 ml of distilled water was added, the solution was allowed to stand at 5°C for 12 hours, and the medium and the cells were removed therefrom using a #2 filter paper (Toyo Roshi Kaisha, Ltd.) to obtain an enzyme extract. The protease activity and the aminopeptidase activity of the enzyme extract were assayed using azocasein and L-leucine-p-nitroanilide, respectively, as a substrate. The results demonstrate that addition of methionine lowered both enzyme activities of both strains, and the activity of the TFM1 strain was higher under all conditions.

### b. Liquid culture in soy flour medium

Liquid culture was carried out using a liquid medium of soybean flour comprising 1.5% defatted soybean powder that had been swollen via heating and pressurization and 1.5% monopotassium dihydrogen phosphate and a medium prepared by adding 10 mM methionine, 20 mM glutamic acid, and 1.5% maltose monohydrate to the aforementioned medium. The aforementioned koji mold conidia (approximately 10⁶ cells) were inoculated on the medium, and rotary shaking culture was carried out at 30°C and 130 rpm for 48 hours. Cells and some insoluble matter in the medium were removed using a #2 filter paper (Toyo Roshi Kaisha , Ltd.) to obtain a culture supernatant. The protease activity and the aminopeptidase activity of this culture supernatant were assayed using azocasein and L-leucine-p-nitroanilide, respectively, as a substrate. The results demonstrate that addition of methionine, glutamine, and maltose lowered both enzyme activities of both strains, and the activity of the TFM1 strain was higher under all conditions. In particular, when methionine, glutamine, and maltose were added, both enzyme activities of the 1764 strains were lowered to a substantially unobservable level, while the activity of the TFM1 strain was as low as approximately half of that when none of these substances had been added.

The above subsections a. and b. demonstrate that the TFM1 strain has an improved capacity for producing extracellular protease and extracellular aminopeptidase.

### Industrial Applicability.

A transcription regulatory factor that regulates the expression of sulfur-assimilatory gene of koji mold and a gene thereof were provided in the present invention. This enables modification of the regulation of the sulfur-assimilatory gene expression of koji mold. Also, it is shown that the enhanced expression of a sulfur-assimilatory gene results in the production of koji mold in which extracellular protease activity and extracellular exopeptidase activity are enhanced. The use of the koji mold according to the present invention can improve the degradation efficiency of a protein-containing substance. Accordingly, the present invention is industrially very useful.

### Free Text of Sequence Listing

SEQ ID NOs: 7, 8, 11, 12 to 24, and 27 to 29 independently represent primer DNA.
SEQ ID NOs: 9 and 10 independently represent adaptor DNA.
SEQ ID NOs: 25 and 26 independently represent probe DNA.

## Claims

1. A protein of the following (a) or (b) or (c):
(a) a protein, which comprises the amino acid sequence as shown in SEQ ID NO: 3;
(b) a protein, which comprises an amino acid sequence derived from the amino acid sequence as shown in SEQ ID NO: 3 by deletion, substitution, or addition of 1 or from 2 to 50 amino acid residues, and which has functions of a transcription factor; or
(c) a protein, which comprises an amino acid sequence having 70% or higher sequence homology to the amino acid sequence as shown in SEQ ID NO: 3 or a fragment thereof, and which protein and fragment has functions of a transcription factor.

2. The protein of claim 1, wherein the deletion, substitution, or addition is of 1 or from 2 to 10 amino acid residues.

3. A transcription factor gene,
(1) which encodes the following protein (a) or (b):
(a) a protein, which comprises the amino acid sequence as shown in SEQ ID NO: 3; or
(b) a protein, which comprises an amino acid sequence derived from the amino acid sequence as shown in SEQ ID NO: 3 by deletion, substitution, or addition of 1 or from 2 to 50 amino acid residues, and which has functions of a transcription factor, or
(2) which comprises DNA, which comprises the nucleotide sequence as shown in SEQ ID NO: 2, or
(3) which encodes the protein according to claim 1, part (c), or
(4) which comprises DNA, which comprises a nucleic acid encoding the amino acid sequence as shown in SEQ ID NO: 3, or
(5) which comprises DNA having 80% or higher sequence homology to DNA comprising the nucleotide sequence as shown in SEQ ID NO: 2, and which encodes a protein having functions of a transcription factor.

4. The gene of claim 3, wherein the deletion, substitution, or addition in the encoded protein is of 1 or from 2 to 10 amino acid residues.

5. A recombinant vector, which comprises the gene according to claim 3 or claim 4.

6. A koji mold,
(1) which comprises the recombinant vector according to claim 5, or
(2) wherein the gene according to claim 3 or claim 4 is expressed in the presence of a low-molecular-weight sulphur-containing substance.

7. The koji mold according to claim 6, which is capable of producing extracellular protease in the presence of a low-molecular-weight sulphur-containing substance.

8. The koji mold according to claim 6, which is capable of producing extracellular protease and extracellular exopeptidase in the presence of a low-molecular-weight sulphur-containing substance.

9. The koji mold according to any one of claims 6-8, wherein the expression of a sulphur-assimilatory gene is derepressed

10. A method for producing protease and/or exopeptidase, which comprises culturing the koji mold according to any one of claims 6-9 in a medium and optionally collecting protease and/or exopeptidase from the resulting culture.

11. The method according to claim 10, wherein the medium comprises 0.5mM or more low-molecular-weight sulphur-containing substance.

12. A method for degrading a protein-containing substance, which comprises degrading a protein-containing substance with the koji mold according to any one of claims 6-9.

13. A method for producing a degradation product of a protein-containing substance, which comprises culturing the koji mold according to any one of claims 6-9, degrading a protein-containing substance with the resulting culture, and optionally collecting a degradiation product of the protein-containing substance therefrom.

## Patentansprüche

1. Protein der folgenden (a) oder (b) oder (c):
(a) ein Protein, das die Aminosäuresequenz umfasst, wie sie in Seq.-ID Nr. 3 dargestellt ist;
(b) ein Protein, das eine Aminosäuresequenz umfasst, die von der Aminosäuresequenz, wie sie in Seq.-ID Nr. 3 dargestellt ist, durch Deletion, Substitution oder Addition von 1 oder von 2 bis 50 Aminosäuresresten abgeleitet ist, und das weiters Funktionen eines Transkriptionsfaktors aufweist; oder
(c) ein Protein, das eine Aminosäuresequenz mit 70 % oder höherer Sequenzhomologie zu der Aminosäuresequenz aufweist, wie sie in Seq.-ID Nr. 3 dargestellt ist, oder ein Fragment davon, wobei das Protein und Fragment Funktionen eines Transkriptionsfaktors aufweist.

2. Protein nach Anspruch 1, worin die Deletion, Substitution oder Addition von 1 oder von 2 bis 10 Aminosäureresten beträgt.

3. Transkriptionsfaktor-Gen, das
(1) für das folgende Protein (a) oder (b) kodiert:
(a) ein Protein, das die Aminosäurensequenz umfasst, wie sie in Seq.-ID Nr. 3 dargestellt ist; oder
(b) ein Protein, das eine Aminosäuresequenz umfasst, die von der Aminosäuresequenz, wie sie in Seq.-ID Nr. 3 dargestellt ist, durch Deletion, Substitution oder Addition von 1 oder von 2 bis 50 Aminosäuresresten abgeleitet ist, und das Funktionen eines Transkriptionsfaktors aufweist; oder
(2) DNA umfasst, welche die Nucleotidsequenz umfasst, wie sie in Seq.-ID Nr. 2 dargestellt ist, oder
(3) für das Protein nach Anspruch 1, Teil (c), kodiert oder
(4) DNA umfasst, die eine Nucleinsäure umfasst, die für die Aminosäuresequenz kodiert, wie sie in Seq.-ID Nr. 3 dargestellt ist, oder
(5) DNA mit 80 % oder höherer Sequenzhomologie zu DNA umfasst, die die Nucleotidsequenz umfasst, wie sie in Seq.-ID Nr. 2 dargestellt ist, und die für ein Protein mit Funktionen eines Transkriptionsfaktors kodiert.

4. Gen nach Anspruch 3, worin die Deletion, Substitution oder Addition im kodierten Protein von 1 oder von 2 bis 10 Aminosäureresten beträgt.

5. Rekombinanter Vektor, umfassend das Gen nach Anspruch 3 oder 4.

6. Koji-Schimmelpilz,
(1) umfassend den rekombinanten Vektor nach Anspruch 5, oder
(2) worin das Gen nach Anspruch 3 oder Anspruch 4 in Gegenwart einer schwefelhältigen Substanz mit niedrigem Molekulargewicht exprimiert wird.

7. Koji-Schimmelpilz nach Anspruch 6, der in der Lage ist, extrazelluläre Protease in Gegenwart einer schwefelhältigen Substanz mit niedrigem Molekulargewicht zu produzieren.

8. Koji-Schimmelpilz nach Anspruch 6, der in der Lage ist, extrazelluläre Protease und extrazelluläre Exopeptidase in Gegenwart einer schwefelhältigen Substanz mit niedrigem Molekulargewicht zu produzieren.

9. Koji-Schimmelpilz nach einem der Ansprüche 6-8, worin die Expression eines schwefelassimilatorischen Gens dereprimiert ist.

10. Verfahren zur Herstellung von Protease und/oder Exopeptidase, umfassend das Züchten des Koji-Schimmelpilzes nach einem der Ansprüche 6-9 in einem Medium und gegebenenfalls das Gewinnen der Protease und/oder der Exopeptidase aus der resultierenden Kultur.

11. Verfahren nach Anspruch 10, worin das Medium 0,5 mM oder mehr einer schwefelhältigen Substanz mit niedrigem Molekulargewicht umfasst.

12. Verfahren zum Abbau einer proteinhältigen Substanz, umfassend den Abbau einer proteinhältigen Substanz mit dem Koji-Schimmelpilz nach einem der Ansprüche 6-9.

13. Verfahren zur Herstellung eines Abbauprodukts einer proteinhältigen Substanz, umfassend das Züchten des Koji-Schimmelpilzes nach einem der Ansprüche 6-9, das Abbauen einer proteinhältigen Substanz mit der resultierenden Kultur sowie gegebenenfalls das Gewinnen eines Abbauprodukts der proteinhältigen Substanz davon.

## Revendications

1. Protéine parmi les suivantes (a) ou (b) ou (c):
(a) une protéine, qui comprend la séquence des acides aminés telle que représentée dans SEQ ID NO: 3;
(b) une protéine, qui comprend une séquence des acides aminés dérivée de la séquence des acides aminés telle que représentée dans SEQ ID NO:3 par suppression, substitution ou addition de 1 ou de 2 à 50 résidus d'acides aminés, et qui a des fonctions d'un facteur de transcription; ou
(c) une protéine, qui comprend la séquence des acides aminés ayant une homologie de séquence de 70% ou plus élevée avec la séquence des acides aminés telle que représentée dans SEQ ID NO:3 ou un fragment de celle-ci, et ladite protéine et fragment a des fonctions d'un facteur de transcription.

2. Protéine selon la revendication 1, dans laquelle la suppression, substitution ou addition est de 1 ou de 2 à 10 résidus d'acide aminé.

3. Gène de facteur de transcription,
(1) qui code pour la protéine suivante (a) ou (b):
(a) une protéine, qui comprend la séquence des acides aminés telle que représentée dans SEQ ID NO:3; ou
(b) une protéine, qui comprend une séquence d'acides aminés dérivée de la séquence d'acides aminés telle que représentée dans SEQ ID NO:3 par suppression, substitution ou addition de 1 ou de 2 à 50 résidus d'acide aminé, et qui a des fonctions d'un facteur de transcription, ou
(2) qui comprend un ADN, qui comprend la séquence de nucléotides comme indiqué dans SEQ ID NO:2, ou
(3) qui code pour la protéine selon la revendication 1, partie (c), ou
(4) qui comprend l'ADN, qui comprend un acide nucléique codant pour la séquence d'acides nucléiques telle qu'indiquée dans la SEQ ID NO:3, ou
(5) qui comprend l'ADN ayant une homologie de séquences de 80% ou plus élevée avec l'ADN comprenant la séquence de nucléotides telle qu'indiquée dans SEQ ID NO:2, et qui code pour une protéine ayant des fonctions d'un facteur de transcription.

4. Gène selon la revendication 3, où la suppression, substitution ou addition dans la protéine codée est de 1 ou de 2 à 10 résidus d'acide aminé.

5. Vecteur recombinant, qui comprend le gène selon la revendication 3 ou la revendication 4.

6. Moisissure koji,
(1) qui comprend le vecteur recombinant selon la revendication 5, ou
(2) où le gène selon la revendication 3 ou la revendication 4 est exprimé en présence d'une substance contenant du soufre d'un poids moléculaire bas.

7. Moisissure koji selon la revendication 6, qui est apte à produire une protéase extracellulaire en présence d'une substance contenant du soufre d'un poids moléculaire bas.

8. Moisissure koji selon la revendication 6, qui est apte à produire une protéase extracellulaire et une exopeptidase extracellulaire en présence d'une substance contenant du soufre d'un poids moléculaire bas.

9. Moisissure koji selon l'une quelconque des revendications 6 à 8, où l'expression d'un gène assimilant le soufre est déréprimée.

10. Méthode de production d'une protéase et/ou exopeptidase, qui comprend la culture de la moisissure koji selon l'une quelconque des revendications 6 à 9 dans un milieu, et en option la collecte de la protéase et/ou de l'exopeptidase de la culture obtenue.

11. Méthode selon la revendication 10, où le milieu comprend 0,5mM ou plus de substance contenant du soufre d'un poids moléculaire bas.

12. Méthode de dégradation d'une substance contenant une protéine, qui comprend la dégradation d'une substance contenant une protéine avec la moisissure koji selon l'une quelconque des revendications 6 à 9.

13. Méthode de production d'un produit de dégradation d'une substance contenant une protéine, qui comprend la culture de la moisissure koji selon l'une quelconque des revendications 6 à 9, la dégradation d'une substance contenant une protéine avec la culture obtenue et en option la collecte d'un produit de dégradation de la substance contenant une protéine de celle-ci.


### SEQUENCE LISTING

<110> NODA INSTITUTE FOR SCIENTIFIC RESEARCH
   NATIONAL INSTITUTE OF ADVANCED INDUSTRIAL SCIENCE AND TECHNOLOGY
<120> TRANSCRIPTION FACTOR, TRANSCRIPTION FACTOR GENE, RECOMBINANT VECTOR COMPRISING TRANSCRIPTION FACTOR GENE, KOJI MOLD TRANSFORMED BY SAID VECTOR, AND USE OF SAID KOJI MOLD
<130> PH-1627-PCT
<140> PCT/JP02/08376
   <141> 2002-08-20
<150> JP 2002-045090
   <151> 2002-02-21
<160> 29
<170> PatentIn. Ver. 2.1
<210> 1
   <211> 3392
   <212> DNA
   <213> Aspergillus oryzae
<220>
   <221> CDS
   <222> (349).. (612)
<220>
   <221> CDS
   <222> (1346).. (1912)
<400> 1
   atctttgcga caaatcatac aatcttggac ctgtgaaatt tgtgcattcc tggaccaatt 60
   ccgagctttc ccctctttga gccgatctgg ggtttgactt tcttctctgg ttcaagcttg 120
   ttctggcgcc atcgccatcc agtctggttc ctgctcttct ccaaagaaga agcagtccaa 180
   aaaaaaatta aattaaatta ggtgggcgca agaggtaatt cagaagaagg aaaaagaaaa 240
   aaaagaaaaa ttaccagaaa cttaaattaa agaggcaagg atcaaaacga ggccattacc 300
   agaacggacg cagctctaaa acctctaaaa acccatcgcg cgagcaat atg tca gat  357
                                                        Met Ser Asp
                                                        1
   gag cac atc gct cgt cag aca gcg tca agc ctc gat agg ctt gag aat   405
   Glu His Ile Ala Arg Gln Thr Ala Ser Ser Leu Asp Arg Leu Glu Asn
       5                   10                  15
   ttc aac ttc tta ctc tcc cga cac gat cct gcc ctg gca aag agt cga   453
   Phe Asn Phe Leu Leu Ser Arg His Asp Pro Ala Leu Ala Lys Ser Arg
   20                  25                  30                  35
   cat tac tct ttc gac gca gac gcg gcc ggc ttg gct cct ttt caa aac   501
   His Tyr Ser Phe Asp Ala Asp Ala Ala Gly Leu Ala Pro Phe Gln Asn
                   40                  45                  50
   ctg agc atg gat tac gac cag act gag gga atg ggc ggt att tcc gtc   549
   Leu Ser Met Asp Tyr Asp Gln Thr Glu Gly Met Gly Gly Ile Ser Val
               55                  60                  65
   agt tcc tac gat agc att gag gat gaa cgg aac ccg atc gat gtg aga   597
   Ser Ser Tyr Asp Ser Ile Glu Asp Glu Arg Asn Pro Ile Asp Val Arg
           70                  75                  80
   ggg tat ccc tat cat ggtaagtctg agcttttatg actctcaatg ttgcattgtt   652
   Gly Tyr Pro Tyr His
       85
   tgcgccatct tctcgatccc tggttcgtgt ctcaattttg tggcctttgc ggcattaggt 712
   ggaaatggta gagctattgc tgaggcgcac tcggatcgtg gccaaagctg atctcgttcg 772
   tctccatcta aatatctcct ttcccttttt ccggggaaaa gcttcttctt gtttgttgtg 832
   ccattagctt gtttgcaatt gttaatcgtg gctgtcattg ttggcgttgc tgtccaactc 892
   ttatcgctcg tggatcactc catactttat tacccataac ctgttaaagt cgaggtttaa 952
   ctttgggctc atgtatgttt tgcttcatct atctccctat tgtttttgcc tgtttttttt 1012
   ttctcttttc tttttttaat ttgccaattt tcttataccc ctttggttgt attctcatct 1072
   gcacccgcat gcctggttga tcacggagac gagcacgcaa tccatcacac caatgacaac 1132
   gtcggtaacc ttgtcgttgg tgttgccgga cccttagcca cgcatctccc tctgttcatc 1192
   taaagctccc caccaagggt cttatgtccc ccttggagct tccctcgtgt gataacactt 1252
   gctccttctt tctgtcttat caccttgcta gatctatgcc tacagcgttg ctgtcctaga 1312
   ttctgctcat tccgttgctt acatctattc cca gca gct gat aaa cac atc aac   1366
                                        Ala Ala Asp Lys His Ile Asn
                                            90                  95
   tac tcc ctc ccc gac caa atg atc tca tat cca gct cac ccg atc tac   1414
   Tyr Ser Leu Pro Asp Gln Met Ile Ser Tyr Pro Ala His Pro Ile Tyr
                   100                 105                 110
   cct cca atc tct tat gga cct gat gat ctg ggt cat gcc ccg ggg gct   1462
   Pro Pro Ile Ser Tyr Gly Pro Asp Asp Leu Gly His Ala Pro Gly Ala
               115                 120                 125
   ctg aca ccc tcg gac gtt tca tca tcc ata tca ccc ccg aat ggt caa   1510
   Leu Thr Pro Ser Asp Val Ser Ser Ser Ile Ser Pro Pro Asn Gly Gln
           130                 135                 140
   ctc gga cac acc aag tac agc acg cag atc cct ggt gat cac ctc gct   1558
   Leu Gly His Thr Lys Tyr Ser Thr Gln Ile Pro Gly Asp His Leu Ala
       145                 150                 155
   tct gca ctt tcg caa gaa gag cat gtt cgt cgt gct gct gaa gaa gac    1606
   Ser Ala Leu Ser Gln Glu Glu His Val Arg Arg Ala Ala Glu Glu Asp
   160                 165                 170                 175
   cgc cgg cgg cga aac acc gcg gct agc gcc cgg ttt cgc atg aaa aag    1654
   Arg Arg Arg Arg Asn Thr Ala Ala Ser Ala Arg Phe Arg Met Lys Lys
                   180                 185                 190
   aag cag cgt gag cag acg ctg gaa cgg aca gtg cga gag act acg gag    1702
   Lys Gln Arg Glu Gln Thr Leu Glu Arg Thr Val Arg Glu Thr Thr Glu
               195                 200                 205
   aag aac gct act ctc gag gcc cgc gta gcc cag ctg gag atg gaa aat    1750
   Lys Asn Ala Thr Leu Glu Ala Arg Val Ala Gln Leu Glu Met Glu Asn
           210                 215                 220
   cga tgg ttg aag aat ctc ttg act gag aaa cac gaa tcg acg agt agt    1798
   Arg Trp Leu Lys Asn Leu Leu Thr Glu Lys His Glu Ser Thr Ser Ser
       225                 230                 235
   cgc atg ccg ccc cca ccg gaa gac agt aca gcc ttg aac caa aaa ggc    1846
   Arg Met Pro Pro Pro Pro Glu Asp Ser Thr Ala Leu Asn Gln Lys Gly
   240                 245                 250                 255
   aac agt ggc gga aac ggc caa aaa cac atc cag cca aaa aag aag ggt    1894
   Asn Ser Gly Gly Asn Gly Gln Lys His Ile Gln Pro Lys Lys Lys Gly
                   260                 265                 270
   gtg ggc acc gat aac tag agactaaaac gaagaagttt tgtttttctg           1942
   Val Gly Thr Asp Asn
               275
   ttaccttgtg tatcttcagc ttttccttct gtctctctcc ctcctctccc tcctctctct 2002
   ccttccgaca ttactacatg ctcatgcttt gggcgttggt tccagtttcc ctcttcgcat 2062
   ctgtcttcat atcctctatc tatttttggg ctggtcatcc gtcctatgat ggaggcactt 2122
   gacgatgtaa tgacctttca tcatttttgt ttctgctgtg cgcgagtgcg agtgcgcgac 2182
   ctgagacacg gtatcatttt ctaggctgtt tggaacgttt ttgttttttt ttccctgttt 2242
   ctcttgctcg cccttctgtt cgttctttct tattagtcat gcttggtgaa tcttgcaggc 2302
   aaattggggg tgatctacag ttttcttgag tcttcacgga tcagggtcag ggtctcctcc 2362
   gccctgtcac ccgcaatcgg agtataagga tgggatttag ttattgtcgg tgttgaattt 2422
   ggcaaaccta tatcgcacgt tggacttaca acatacgagc ctaccgtgcc cgcagtagct 2482
   cggcttaaat ctctgatagg gactagagct gagaatcaac tgattggcgt tcaaatttca 2542
   ttcatccgaa gtttgtccta aacccctccg cgtatagatt gcaggcgaga ggtatacaga 2602
   atatatgaat cacacacagg atccaggaca caggcacatt cgagcaaatt gggtttgtgt 2662
   cttgaaatat atatggggag tgttttcttg aacatacatt cgcaagggac tcggagctca 2722
   tcaggctgca atgtttgcct tgctcttttt ttatcctagg gctgttctat cttggcgctt 2782
   gttacggagt actggctata cgttttgaat agtgactaca tacatatcag gcttcagctt 2842
   ttcatctttg gacaagagat ccaaccgcaa aaacagtatt gtactctact acaatccacg 2902
   ggtcgccttt gtaagtctct gaaaagaaaa gaaaaagaaa agaaaagtac cgtagggagc 2962
   agatatgtcc tctagaatca ataaaaacaa aacaccccca gaagtgatca tcaccctcga 3022
   ccgaagaaaa gcggtcggtc gaaagtgtag aatccgggga acaacaaaag caacgaatgg 3082
   agcatgggcg ttgacatcat actcgtcggc tattcatggt ccagaaggaa gttccggggc 3142
   ggctaacgat aatcagtacc gaaacagtcc acgatcacgg ggacgtacgg cgatagttga 3202
   gtttcgagtt gtctcgagtt gaaatagtca cgggattggc atttccatat gatacctgcc 3262
   cgggcggccg ctcgagccct atagtgagtc gtattaggat ggaatccata tgactagtag 3322
   atcctctaga gtcgacctgc aggcatgcaa gctttcccta tagtgagtcg tattagagct 3382
   tggcgtaatc 3392
<210> 2
   <211> 831
   <212> DNA
   <213> Aspergillus oryzae
<220>
   <221> CDS
   <222> (1)..(831)
<400> 2
   atg tca gat gag cac atc gct cgt cag aca gcg tca agc ctc gat agg    48
   Met Ser Asp Glu His Ile Ala Arg Gln Thr Ala Ser Ser Leu Asp Arg
    1               5                  10                  15
   ctt gag aat ttc aac ttc tta ctc tcc cga cac gat cct gcc ctg gca    96
   Leu Glu Asn Phe Asn Phe Leu Leu Ser Arg His Asp Pro Ala Leu Ala
               20                  25                  30
   aag agt cga cat tac tct ttc gac gca gac gcg gcc ggc ttg gct cct   144
   Lys Ser Arg His Tyr Ser Phe Asp Ala Asp Ala Ala Gly Leu Ala Pro
           35                  40                  45
   ttt caa aac ctg agc atg gat tac gac cag act gag gga atg ggc ggt   192
   Phe Gln Asn Leu Ser Met Asp Tyr Asp Gln Thr Glu Gly Met Gly Gly
       50                  55                  60
   att tcc gtc agt tcc tac gat agc att gag gat gaa cgg aac ccg atc   240
   Ile Ser Val Ser Ser Tyr Asp Ser Ile Glu Asp Glu Arg Asn Pro Ile
   65                  70                  75                  80
   gat gtg aga ggg tat ccc tat cat gca gct gat aaa cac atc aac tac   288
   Asp Val Arg Gly Tyr Pro Tyr His Ala Ala Asp Lys His Ile Asn Tyr
                   85                  90                  95
   tcc ctc ccc gac caa atg atc tca tat cca gct cac ccg atc tac cct   336
   Ser Leu Pro Asp Gln Met Ile Ser Tyr Pro Ala His Pro Ile Tyr Pro
               100                 105                 110
   cca atc tct tat gga cct gat gat ctg ggt cat gcc ccg ggg gct ctg   384
   Pro Ile Ser Tyr Gly Pro Asp Asp Leu Gly His Ala Pro Gly Ala Leu
           115                 120                 125
   aca ccc tcg gac gtt tca tca tcc ata tca ccc ccg aat ggt caa ctc   432
   Thr Pro Ser Asp Val Ser Ser Ser Ile Ser Pro Pro Asn Gly Gln Leu
       130                 135                 140
   gga cac acc aag tac agc acg cag atc cct ggt gat cac ctc gct tct 480
   Gly His Thr Lys Tyr Ser Thr Gln Ile Pro Gly Asp His Leu Ala Ser
   145 150 155 160
   gca ctt tcg caa gaa gag cat gtt cgt cgt gct gct gaa gaa gac cgc 528
   Ala Leu Ser Gln Glu Glu His Val Arg Arg Ala Ala Glu Glu Asp Arg
   165 170 175
   cgg cgg cga aac acc gcg gct agc gcc cgg ttt cgc atg aaa aag aag 576
   Arg Arg Arg Asn Thr Ala Ala Ser Ala Arg Phe Arg Met Lys Lys Lys
   180 185 190
   cag cgt gag cag acg ctg gaa cgg aca gtg cga gag act acg gag aag 624
   Gln Arg Glu Gln Thr Leu Glu Arg Thr Val Arg Glu Thr Thr Glu Lys
   195 200 205
   aac gct act ctc gag gcc cgc gta gcc cag ctg gag atg gaa aat cga 672
   Asn Ala Thr Leu Glu Ala Arg Val Ala Gln Leu Glu Met Glu Asn Arg
   210 215 220
   tgg ttg aag aat ctc ttg act gag aaa cac gaa tcg acg agt agt cgc 720
   Trp Leu Lys Asn Leu Leu Thr Glu Lys His Glu Ser Thr Ser Ser Arg
   225 230 235 240
   atg ccg ccc cca ccg gaa gac agt aca gcc ttg aac caa aaa ggc aac 768
   Met Pro Pro Pro Pro Glu Asp Ser Thr Ala Leu Asn Gln Lys Gly Asn
   245 250 255
   agt ggc gga aac ggc caa aaa cac atc cag cca aaa aag aag ggt gtg 816
   Ser Gly Gly Asn Gly Gln Lys His Ile Gln Pro Lys Lys Lys Gly Val
   260 265 270
   ggc acc gat aac tag 831
   Gly Thr Asp Asn
   275
<210> 3
<211> 276
   <212> PRT
   <213> Aspergillus oryzae
<400> 3
   Met Ser Asp Glu His Ile Ala Arg Gln Thr Ala Ser Ser Leu Asp Arg
   1               5                   10                  15
   Leu Glu Asn Phe Asn Phe Leu Leu Ser Arg His Asp Pro Ala Leu Ala
               20                  25                  30
   Lys Ser Arg His Tyr Ser Phe Asp Ala Asp Ala Ala Gly Leu Ala Pro
           35                  40                  45
   Phe Gln Asn Leu Ser Met Asp Tyr Asp Gln Thr Glu Gly Met Gly Gly
       50                  55                  60
   Ile Ser Val Ser Ser Tyr Asp Ser Ile Glu Asp Glu Arg Asn Pro Ile
   65                  70                  75                  80
   Asp Val Arg Gly Tyr Pro Tyr His Ala Ala Asp Lys His Ile Asn Tyr
                   85                  90                  95
   Ser Leu Pro Asp Gln Met Ile Ser Tyr Pro Ala His Pro Ile Tyr Pro
               100                 105                 110
   Pro Ile Ser Tyr Gly Pro Asp Asp Leu Gly His Ala Pro Gly Ala Leu
           115                 120                 125
   Thr Pro Ser Asp Val Ser Ser Ser Ile Ser Pro Pro Asn Gly Gln Leu
       130                 135                 140
   Gly His Thr Lys Tyr Ser Thr Gln Ile Pro Gly Asp His Leu Ala Ser
   145                 150                 155                 160
   Ala Leu Ser Gln Glu Glu His Val Arg Arg Ala Ala Glu Glu Asp Arg
                   165                 170                 175
   Arg Arg Arg Asn Thr Ala Ala Ser Ala Arg Phe Arg Met Lys Lys Lys
               180                 185                 190
   Gln Arg Glu Gln Thr Leu Glu Arg Thr Val Arg Glu Thr Thr Glu Lys
   195 200 205
   Asn Ala Thr Leu Glu Ala Arg Val Ala Gln Leu Glu Met Glu Asn Arg
   210 215 220
   Trp Leu Lys Asn Leu Leu Thr Glu Lys His Glu Ser Thr Ser Ser Arg
   225 230 235 240
   Met Pro Pro Pro Pro Glu Asp Ser Thr Ala Leu Asn Gln Lys Gly Asn
   245 250 255
   Ser Gly Gly Asn Gly Gln Lys His Ile Gln Pro Lys Lys Lys Gly Val
   260 265 270
   Gly Thr Asp Asn
   275
<210> 4
   <211> 2660
   <212> DNA
   <213> Aspergillus oryzae
<220>
   <221> CDS
   <222> (443)..(814)
<220>
   <221> CDS
   <222> (864)..(2546)
<400> 4
   atcttttctt tcgggcgcat ccttctgcac gtgtttcgaa tttggctgga ccattgaaga 60
   cagaaagaag aagaccctac tcctctcttc catacaccct ctctctgtcg ctttcgcttt 120
   ccccatcccc gcctatcaac cactaaatct ccttatcgcg cgacctatca atccccgacg 180
   gtcgccactg tcactcccct atcggcgcac aaccggatac cgcagtattg actttagact 240
   tcaacaaacc ttgaagaagg tagtcgctgg cgtgatattt cgtcgtcttc tcgattttct 300
   caaggttctt cattaccttc tcgggacgac tgctaccacg gtagcgcgcg ggcggtagga 360
   acctcgttcc agtcacacga tgcagttcga cgaccgatca gtgcgtgaag gtagcgactc 420
   ttcgcagact ttcttgatga aa atg gcc caa ccg acc gga gaa ctt aca cat  472
                            Met Ala Gln Pro Thr Gly Glu Leu Thr His
                            1               5                   10
   cca tcg caa caa caa caa cta caa cta caa cag cag ttc ttc cag tct   520
   Pro Ser Gln Gln Gln Gln Leu Gln Leu Gln Gln Gln Phe Phe Gln Ser
               15                  20                  25
   atc ttt ggc ggc gca tcg gac acc act gag gag att gac acc gag acc   568
   Ile Phe Gly Gly Ala Ser Asp Thr Thr Glu Glu Ile Asp Thr Glu Thr
           30                  35                  40
   gat tct aac cat cga agg ccc cac agt ttc ggt gcc gct gcg acg act   616
   Asp Ser Asn His Arg Arg Pro His Ser Phe Gly Ala Ala Ala Thr Thr
       45                  50                  55
   ccc gca aag ctt gca aac aag aat gtc gca cca ttt ctc gtc aaa cac   664
   Pro Ala Lys Leu Ala Asn Lys Asn Val Ala Pro Phe Leu Val Lys His
       60                  65                  70
   atc ccc gaa caa tac ggt ccc ttg ggg tcg cga aga acc gac aag ttg   712
   Ile Pro Glu Gln Tyr Gly Pro Leu Gly Ser Arg Arg Thr Asp Lys Leu
   75                  80                  85                  90
   gag gat ttg agc agc ccg aac tcg aag ttt tgc tat cgc cac cgg cca   760
   Glu Asp Leu Ser Ser Pro Asn Ser Lys Phe Cys Tyr Arg His Arg Pro
                   95                  100                 105
   gac ctt aaa tgc aga cga cag gca gac gaa ccg tcc atg gat aaa cta   808
   Asp Leu Lys Cys Arg Arg Gln Ala Asp Glu Pro Ser Met Asp Lys Leu
               110                 115                 120
   cag agg gtagggtttc ttagggcttg tcatgtttct cttagctaat gggtcatag gaa 866
   Gln Arg Glu
   125
   ttg gaa acg ctg cct ccc agc gac caa caa ggc att gcc cat gta tgg 914
   Leu Glu Thr Leu Pro Pro Ser Asp Gln Gln Gly Ile Ala His Val Trp
   130 135 140
   tct ctg ttt tcg gcc gct ccg gcc aag cac cgc aaa ttg atc ctc cag 962
   Ser Leu Phe Ser Ala Ala Pro Ala Lys His Arg Lys Leu Ile Leu Gln
   145 150 155
   ggg atc atg gct cag tgc tgt ttc ccg caa ctt tcg ttc gtg tcc gct 1010
   Gly Ile Met Ala Gln Cys Cys Phe Pro Gln Leu Ser Phe Val Ser Ala
   160 165 170
   acc gtt cga gac ctc atc cga atc gac ttt ctg acc gct ctt cca ccg 1058
   Thr Val Arg Asp Leu Ile Arg Ile Asp Phe Leu Thr Ala Leu Pro Pro
   175 180 185
   gag atc tcg ttc aaa att ctg tgc tac ctc gac acc acc tcg ctg tgc 1106
   Glu Ile Ser Phe Lys Ile Leu Cys Tyr Leu Asp Thr Thr Ser Leu Cys
   190 195 200 205
   aaa gcc gcc cag gtg tcc agc cgc tgg cgg gca ctg gca gat gat gat 1154
   Lys Ala Ala Gln Val Ser Ser Arg Trp Arg Ala Leu Ala Asp Asp Asp
   210 215 220
   gtg gtt tgg cat cgg atg tgc gaa caa cac atc cac cgc aaa tgc aag 1202
   Val Val Trp His Arg Met Cys Glu Gln His Ile His Arg Lys Cys Lys
   225 230 235
   aaa tgt ggc tgg ggg ctt cct ctt ctg gag cgg aaa cgt ctg cgg gag 1250
   Lys Cys Gly Trp Gly Leu Pro Leu Leu Glu Arg Lys Arg Leu Arg Glu
   240 245 250
   tcc aag cgc gaa att gaa ttg cgt gcc acc acc tgg gac gtc agc gga 1298
   Ser Lys Arg Glu Ile Glu Leu Arg Ala Thr Thr Trp Asp Val Ser Gly
   255 260 265
   ccc gcg cag aac gca gga ggt gcc gag tgc agt gca cca cat gcg gac 1346
   Pro Ala Gln Asn Ala Gly Gly Ala Glu Cys Ser Ala Pro His Ala Asp
   270 275 280 285
   gat gtg att acc cag aaa cgg aag gcg gat tcg agc gac gat gag acg 1394
   Asp Val Ile Thr Gln Lys Arg Lys Ala Asp Ser Ser Asp Asp Glu Thr
   290 295 300
   gca atc gtg aag cgg cat tgt tcc tcg ctg gat gct cga ccg gag cca 1442
   Ala Ile Val Lys Arg His Cys Ser Ser Leu Asp Ala Arg Pro Glu Pro
   305 310 315
   gat gag gat tac tat acg act cgg tat cgg ccg tgg aag gag gtc tac 1490
   Asp Glu Asp Tyr Tyr Thr Thr Arg Tyr Arg Pro Trp Lys Glu Val Tyr
   320 325 330
   aag gac cga ttc aag gtc ggc acg aat tgg aaa tac ggc cgg tgt tcc 1538
   Lys Asp Arg Phe Lys Val Gly Thr Asn Trp Lys Tyr Gly Arg Cys Ser
   335 340 345
   acc aag gtg ttc aaa ggc cac acc aac gga gtg atg tgc ttg caa ttc 1586
   Thr Lys Val Phe Lys Gly His Thr Asn Gly Val Met Cys Leu Gln Phe
   350 355 360 365
   gag gac aac att ttg gcg act ggt tca tac gac gcg acc atc aag att 1634
   Glu Asp Asn Ile Leu Ala Thr Gly Ser Tyr Asp Ala Thr Ile Lys Ile
   370 375 380
   tgg gat act gaa acc gga gaa gag ctg cgt acc cta cgc gga cac caa 1682
   Trp Asp Thr Glu Thr Gly Glu Glu Leu Arg Thr Leu Arg Gly His Gln
   385 390 395
   tcc ggt att cgc tgt ctc cag ttc gac gac aca aaa ttg atc agc ggc 1730
   Ser Gly Ile Arg Cys Leu Gln Phe Asp Asp Thr Lys Leu Ile Ser Gly
   400 405 410
   agt atg gac cgc agc ttg aag gtg tgg aat tgg cgt acg ggc gag tgc 1778
   Ser Met Asp Arg Ser Leu Lys Val Trp Asn Trp Arg Thr Gly Glu Cys
   415 420 425
   att tcc acc tac acg ggt cac cgt ggt gga gtg atc gga ctg cac ttt 1826
   Ile Ser Thr Tyr Thr Gly His Arg Gly Gly Val Ile Gly Leu His Phe
   430 435 440 445
   gat gca acg att ctt gcc tca gcc tcc gtt gat aag acg gtc aag att 1874
   Asp Ala Thr Ile Leu Ala Ser Ala Ser Val Asp Lys Thr Val Lys Ile
   450 455 460
   tgg aac ttc gag gat aag tcg aca ttt ctc ctg cgc gga cac acc gat 1922
   Trp Asn Phe Glu Asp Lys Ser Thr Phe Leu Leu Arg Gly His Thr Asp
   465 470 475
   tgg gtc aac gcg gtc cga gtg gac acg act tct cga acg gtg ttc tcg 1970
   Trp Val Asn Ala Val Arg Val Asp Thr Thr Ser Arg Thr Val Phe Ser
   480 485 490
   gct tcg gac gac tgc acc gtt cgc ttg tgg gac ttg gac acc aag gca 2018
   Ala Ser Asp Asp Cys Thr Val Arg Leu Trp Asp Leu Asp Thr Lys Ala
   495 500 505
   tgt ctc cgg acc ttc cat ggc cat gtc ggc caa gtc caa cag gtg gtt 2066
   Cys Leu Arg Thr Phe His Gly His Val Gly Gln Val Gln Gln Val Val
   510 515 520 525
   cct ttg ccc cgg gag ttc gag ttc gaa gac cac gac gct gaa tgt gat 2114
   Pro Leu Pro Arg Glu Phe Glu Phe Glu Asp His Asp Ala Glu Cys Asp
   530 535 540
   aac gat aac atg agc acc aca tct gga gac acg gaa tca aac tcc ctt 2162
   Asn Asp Asn Met Ser Thr Thr Ser Gly Asp Thr Glu Ser Asn Ser Leu
   545 550 555
   cag gca acg ctc gga ctg gag tcg aac gcc act gag acg tct gtc ttt 2210
   Gln Ala Thr Leu Gly Leu Glu Ser Asn Ala Thr Glu Thr Ser Val Phe
   560 565 570
   ggc ccc tca ttt gac aat ggc cgc cca gcc cca cca cgc tac att gtc    2258
   Gly Pro Ser Phe Asp Asn Gly Arg Pro Ala Pro Pro Arg Tyr Ile Val
       575                 580                 585
   acg agc gcc tta gat tcc acc att cgt ctc tgg gag acc acg acc ggt    2306
   Thr Ser Ala Leu Asp Ser Thr Ile Arg Leu Trp Glu Thr Thr Thr Gly
   590                 595                 600                 605
   cgc tgc ctg cgt acc ttc ttc ggt cac ctc gag ggc gtt tgg gcg ctc    2354
   Arg Cys Leu Arg Thr Phe Phe Gly His Leu Glu Gly Val Trp Ala Leu
                   610                 615                 620
   ggt gct gat act ctc cga atc gtg tcc ggt gcg gaa gac cgg atg gtc    2402
   Gly Ala Asp Thr Leu Arg Ile Val Ser Gly Ala Glu Asp Arg Met Val
               625                 630                 635
   aag atc tgg gat ccc aga acc ggt aaa tgc gag cgc acc ttc acc ggc    2450
   Lys Ile Trp Asp Pro Arg Thr Gly Lys Cys Glu Arg Thr Phe Thr Gly
           640                 645                 650
   cat tcc ggc cca gtg acc tgt atc ggt ctc ggc gac agt cgg ttt gcg    2498
   His Ser Gly Pro Val Thr Cys Ile Gly Leu Gly Asp Ser Arg Phe Ala
       655                 660                 665
   acc ggc agt gag gat tgc gaa gtg cgc atg tac agt ttc cgc aat taa    2546
   Thr Gly Ser Glu Asp Cys Glu Val Arg Met Tyr Ser Phe Arg Asn
   670                 675                 680                 685
   atattctgcc ttcttttttc ttcttctcgt ggcccgtctc ctgtggagac atgatgcgcc 2606
   tatgtttaat gtgctttggt tacaaatacc cttcaagttg gcgctagggt cagg       2660
<210> 5
   <211> 2055
   <212> DNA
   <213> Aspergillus oryzae
<220>
   <221> CDS
   <222> (1)..(2055)
<400> 5
   atg gcc caa ccg acc gga gaa ctt aca cat cca tcg caa caa caa caa    48
   Met Ala Gln Pro Thr Gly Glu Leu Thr His Pro Ser Gln Gln Gln Gln
    1               5                  10                  15
   cta caa cta caa cag cag ttc ttc cag tct atc ttt ggc ggc gca tcg    96
   Leu Gln Leu Gln Gln Gln Phe Phe Gln Ser Ile Phe Gly Gly Ala Ser
               20                  25                  30
   gac acc act gag gag att gac acc gag acc gat tct aac cat cga agg   144
   Asp Thr Thr Glu Glu Ile Asp Thr Glu Thr Asp Ser Asn His Arg Arg
           35                  40                  45
   ccc cac agt ttc ggt gcc gct gcg acg act ccc gca aag ctt gca aac   192
   Pro His Ser Phe Gly Ala Ala Ala Thr Thr Pro Ala Lys Leu Ala Asn
       50                  55                  60
   aag aat gtc gca cca ttt ctc gtc aaa cac atc ccc gaa caa tac ggt   240
   Lys Asn Val Ala Pro Phe Leu Val Lys His Ile Pro Glu Gln Tyr Gly
   65                  70                  75                  80
   ccc ttg ggg tcg cga aga acc gac aag ttg gag gat ttg agc agc ccg   288
   Pro Leu Gly Ser Arg Arg Thr Asp Lys Leu Glu Asp Leu Ser Ser Pro
                   85                  90                  95
   aac tcg aag ttt tgc tat cgc cac cgg cca gac ctt aaa tgc aga cga   336
   Asn Ser Lys Phe Cys Tyr Arg His Arg Pro Asp Leu Lys Cys Arg Arg
               100                 105                 110
   cag gca gac gaa ccg tcc atg gat aaa cta cag agg gaa ttg gaa acg   384
   Gln Ala Asp Glu Pro Ser Met Asp Lys Leu Gln Arg Glu Leu Glu Thr
           115                 120                 125
   ctg cct ccc agc gac caa caa ggc att gcc cat gta tgg tct ctg ttt 432
   Leu Pro Pro Ser Asp Gln Gln Gly Ile Ala His Val Trp Ser Leu Phe
   130 135 140
   tcg gcc gct ccg gcc aag cac cgc aaa ttg atc ctc cag ggg atc atg 480
   Ser Ala Ala Pro Ala Lys His Arg Lys Leu Ile Leu Gln Gly Ile Met
   145 150 155 160
   gct cag tgc tgt ttc ccg caa ctt tcg ttc gtg tcc gct acc gtt cga 528
   Ala Gln Cys Cys Phe Pro Gln Leu Ser Phe Val Ser Ala Thr Val Arg
   165 170 175
   gac ctc atc cga atc gac ttt ctg acc gct ctt cca ccg gag atc tcg 576
   Asp Leu Ile Arg Ile Asp Phe Leu Thr Ala Leu Pro Pro Glu Ile Ser
   180 185 190
   ttc aaa att ctg tgc tac ctc gac acc acc tcg ctg tgc aaa gcc gcc 624
   Phe Lys Ile Leu Cys Tyr Leu Asp Thr Thr Ser Leu Cys Lys Ala Ala
   195 200 205
   cag gtg tcc agc cgc tgg cgg gca ctg gca gat gat gat gtg gtt tgg 672
   Gln Val Ser Ser Arg Trp Arg Ala Leu Ala Asp Asp Asp Val Val Trp
   210 215 220
   cat cgg atg tgc gaa caa cac atc cac cgc aaa tgc aag aaa tgt ggc 720
   His Arg Met Cys Glu Gln His Ile His Arg Lys Cys Lys Lys Cys Gly
   225 230 235 240
   tgg ggg ctt cct ctt ctg gag cgg aaa cgt ctg cgg gag tcc aag cgc 768
   Trp Gly Leu Pro Leu Leu Glu Arg Lys Arg Leu Arg Glu Ser Lys Arg
   245 250 255
   gaa att gaa ttg cgt gcc acc acc tgg gac gtc agc gga ccc gcg cag 816
   Glu Ile Glu Leu Arg Ala Thr Thr Trp Asp Val Ser Gly Pro Ala Gln
   260 265 270
   aac gca gga ggt gcc gag tgc agt gca cca cat gcg gac gat gtg att 864
   Asn Ala Gly Gly Ala Glu Cys Ser Ala Pro His Ala Asp Asp Val Ile
   275 280 285
   acc cag aaa cgg aag gcg gat tcg agc gac gat gag acg gca atc gtg 912
   Thr Gln Lys Arg Lys Ala Asp Ser Ser Asp Asp Glu Thr Ala Ile Val
   290 295 300
   aag cgg cat tgt tcc tcg ctg gat gct cga ccg gag cca gat gag gat 960
   Lys Arg His Cys Ser Ser Leu Asp Ala Arg Pro Glu Pro Asp Glu Asp
   305 310 315 320
   tac tat acg act cgg tat cgg ccg tgg aag gag gtc tac aag gac cga 1008
   Tyr Tyr Thr Thr Arg Tyr Arg Pro Trp Lys Glu Val Tyr Lys Asp Arg
   325 330 335
   ttc aag gtc ggc acg aat tgg aaa tac ggc cgg tgt tcc acc aag gtg 1056
   Phe Lys Val Gly Thr Asn Trp Lys Tyr Gly Arg Cys Ser Thr Lys Val
   340 345 350
   ttc aaa ggc cac acc aac gga gtg atg tgc ttg caa ttc gag gac aac 1104
   Phe Lys Gly His Thr Asn Gly Val Met Cys Leu Gln Phe Glu Asp Asn
   355 360 365
   att ttg gcg act ggt tca tac gac gcg acc atc aag att tgg gat act 1152
   Ile Leu Ala Thr Gly Ser Tyr Asp Ala Thr Ile Lys Ile Trp Asp Thr
   370 375 380
   gaa acc gga gaa gag ctg cgt acc cta cgc gga cac caa tcc ggt att 1200
   Glu Thr Gly Glu Glu Leu Arg Thr Leu Arg Gly His Gln Ser Gly Ile
   385 390 395 400
   cgc tgt ctc cag ttc gac gac aca aaa ttg atc agc ggc agt atg gac 1248
   Arg Cys Leu Gln Phe Asp Asp Thr Lys Leu Ile Ser Gly Ser Met Asp
   405 410 415
   cgc agc ttg aag gtg tgg aat tgg cgt acg ggc gag tgc att tcc acc 1296
   Arg Ser Leu Lys Val Trp Asn Trp Arg Thr Gly Glu Cys Ile Ser Thr
   420 425 430
   tac acg ggt cac cgt ggt gga gtg atc gga ctg cac ttt gat gca acg 1344
   Tyr Thr Gly His Arg Gly Gly Val Ile Gly Leu His Phe Asp Ala Thr
           435                 440                 445
   att ctt gcc tca gcc tcc gtt gat aag acg gtc aag att tgg aac ttc    1392
   Ile Leu Ala Ser Ala Ser Val Asp Lys Thr Val Lys Ile Trp Asn Phe
       450                 455                 460
   gag gat aag tcg aca ttt ctc ctg cgc gga cac acc gat tgg gtc aac    1440
   Glu Asp Lys Ser Thr Phe Leu Leu Arg Gly His Thr Asp Trp Val Asn
   465                 470                 475                 480
   gcg gtc cga gtg gac acg act tct cga acg gtg ttc tcg gct tcg gac    1488
   Ala Val Arg Val Asp Thr Thr Ser Arg Thr Val Phe Ser Ala Ser Asp
                   485                 490                 495
   gac tgc acc gtt cgc ttg tgg gac ttg gac acc aag gca tgt ctc cgg    1536
   Asp Cys Thr Val Arg Leu Trp Asp Leu Asp Thr Lys Ala Cys Leu Arg
               500                 505                 510
   acc ttc cat ggc cat gtc ggc caa gtc caa cag gtg gtt cct ttg ccc    1584
   Thr Phe His Gly His Val Gly Gln Val Gln Gln Val Val Pro Leu Pro
           515                 520                 525
   cgg gag ttc gag ttc gaa gac cac gac gct gaa tgt gat aac gat aac    1632
   Arg Glu Phe Glu Phe Glu Asp His Asp Ala Glu Cys Asp Asn Asp Asn
       530                 535                 540
   atg agc acc aca tct gga gac acg gaa tca aac tcc ctt cag gca acg    1680
   Met Ser Thr Thr Ser Gly Asp Thr Glu Ser Asn Ser Leu Gln Ala Thr
   545                 550                 555                 560
   ctc gga ctg gag tcg aac gcc act gag acg tct gtc ttt ggc ccc tca    1728
   Leu Gly Leu Glu Ser Asn Ala Thr Glu Thr Ser Val Phe Gly Pro Ser
                   565                 570                 575
   ttt gac aat ggc cgc cca gcc cca cca cgc tac att gtc acg agc gcc    1776
   Phe Asp Asn Gly Arg Pro Ala Pro Pro Arg Tyr Ile Val Thr Ser Ala
               580                 585                 590
   tta gat tcc acc att cgt ctc tgg gag acc acg acc ggt cgc tgc ctg 1824
   Leu Asp Ser Thr Ile Arg Leu Trp Glu Thr Thr Thr Gly Arg Cys Leu
   595 600 605
   cgt acc ttc ttc ggt cac ctc gag ggc gtt tgg gcg ctc ggt gct gat 1872
   Arg Thr Phe Phe Gly His Leu Glu Gly Val Trp Ala Leu Gly Ala Asp
   610 615 620
   act ctc cga atc gtg tcc ggt gcg gaa gac cgg atg gtc aag atc tgg 1920
   Thr Leu Arg Ile Val Ser Gly Ala Glu Asp Arg Met Val Lys Ile Trp
   625 630 635 640
   gat ccc aga acc ggt aaa tgc gag cgc acc ttc acc ggc cat tcc ggc 1968
   Asp Pro Arg Thr Gly Lys Cys Glu Arg Thr Phe Thr Gly His Ser Gly
   645 650 655
   cca gtg acc tgt atc ggt ctc ggc gac agt cgg ttt gcg acc ggc agt 2016
   Pro Val Thr Cys Ile Gly Leu Gly Asp Ser Arg Phe Ala Thr Gly Ser
   660 665 670
   gag gat tgc gaa gtg cgc atg tac agt ttc cgc aat taa 2055
   Glu Asp Cys Glu Val Arg Met Tyr Ser Phe Arg Asn
   675 680 685
<210> 6
   <211> 684
   <212> PRT
   <213> Aspergillus oryzae
<400> 6
   Met Ala Gln Pro Thr Gly Glu Leu Thr His Pro Ser Gln Gln Gln Gln
   1 5 10 15
   Leu Gln Leu Gln Gln Gln Phe Phe Gln Ser Ile Phe Gly Gly Ala Ser
   20 25 30
   Asp Thr Thr Glu Glu Ile Asp Thr Glu Thr Asp Ser Asn His Arg Arg
   35 40 45
   Pro His Ser Phe Gly Ala Ala Ala Thr Thr Pro Ala Lys Leu Ala Asn
   50 55 60
   Lys Asn Val Ala Pro Phe Leu Val Lys His Ile Pro Glu Gln Tyr Gly
   65 70 75 80
   Pro Leu Gly Ser Arg Arg Thr Asp Lys Leu Glu Asp Leu Ser Ser Pro
   85 90 95
   Asn Ser Lys Phe Cys Tyr Arg His Arg Pro Asp Leu Lys Cys Arg Arg
   100 105 110
   Gln Ala Asp Glu Pro Ser Met Asp Lys Leu Gln Arg Glu Leu Glu Thr
   115 120 125
   Leu Pro Pro Ser Asp Gln Gln Gly Ile Ala His Val Trp Ser Leu Phe
   130 135 140
   Ser Ala Ala Pro Ala Lys His Arg Lys Leu Ile Leu Gln Gly Ile Met
   145 150 155 160
   Ala Gln Cys Cys Phe Pro Gln Leu Ser Phe Val Ser Ala Thr Val Arg
   165 170 175
   Asp Leu Ile Arg Ile Asp Phe Leu Thr Ala Leu Pro Pro Glu Ile Ser
   180 185 190
   Phe Lys Ile Leu Cys Tyr Leu Asp Thr Thr Ser Leu Cys Lys Ala Ala
   195 200 205
   Gln Val Ser Ser Arg Trp Arg Ala Leu Ala Asp Asp Asp Val Val Trp
   210 215 220
   His Arg Met Cys Glu Gln His Ile His Arg Lys Cys Lys Lys Cys Gly
   225 230 235 240
   Trp Gly Leu Pro Leu Leu Glu Arg Lys Arg Leu Arg Glu Ser Lys Arg
   245 250 255
   Glu Ile Glu Leu Arg Ala Thr Thr Trp Asp Val Ser Gly Pro Ala Gln
   260 265 270
   Asn Ala Gly Gly Ala Glu Cys Ser Ala Pro His Ala Asp Asp Val Ile
   275 280 285
   Thr Gln Lys Arg Lys Ala Asp Ser Ser Asp Asp Glu Thr Ala Ile Val
   290 295 300
   Lys Arg His Cys Ser Ser Leu Asp Ala Arg Pro Glu Pro Asp Glu Asp
   305 310 315 320
   Tyr Tyr Thr Thr Arg Tyr Arg Pro Trp Lys Glu Val Tyr Lys Asp Arg
   325 330 335
   Phe Lys Val Gly Thr Asn Trp Lys Tyr Gly Arg Cys Ser Thr Lys Val
   340 345 350
   Phe Lys Gly His Thr Asn Gly Val Met Cys Leu Gln Phe Glu Asp Asn
   355 360 365
   Ile Leu Ala Thr Gly Ser Tyr Asp Ala Thr Ile Lys Ile Trp Asp Thr
   370 375 380
   Glu Thr Gly Glu Glu Leu Arg Thr Leu Arg Gly His Gln Ser Gly Ile
   385 390 395 400
   Arg Cys Leu Gln Phe Asp Asp Thr Lys Leu Ile Ser Gly Ser Met Asp
   405 410 415
   Arg Ser Leu Lys Val Trp Asn Trp Arg Thr Gly Glu Cys Ile Ser Thr
   420 425 430
   Tyr Thr Gly His Arg Gly Gly Val Ile Gly Leu His Phe Asp Ala Thr
   435 440 445
   Ile Leu Ala Ser Ala Ser Val Asp Lys Thr Val Lys Ile Trp Asn Phe
   450 455 460
   Glu Asp Lys Ser Thr Phe Leu Leu Arg Gly His Thr Asp Trp Val Asn
   465 470 475 480
   Ala Val Arg Val Asp Thr Thr Ser Arg Thr Val Phe Ser Ala Ser Asp
   485 490 495
   Asp Cys Thr Val Arg Leu Trp Asp Leu Asp Thr Lys Ala Cys Leu Arg
   500 505 510
   Thr Phe His Gly His Val Gly Gln Val Gln Gln Val Val Pro Leu Pro
   515 520 525
   Arg Glu Phe Glu Phe Glu Asp His Asp Ala Glu Cys Asp Asn Asp Asn
   530 535 540
   Met Ser Thr Thr Ser Gly Asp Thr Glu Ser Asn Ser Leu Gln Ala Thr
   545 550 555 560
   Leu Gly Leu Glu Ser Asn Ala Thr Glu Thr Ser Val Phe Gly Pro Ser
   565 570 575
   Phe Asp Asn Gly Arg Pro Ala Pro Pro Arg Tyr Ile Val Thr Ser Ala
   580 585 590
   Leu Asp Ser Thr Ile Arg Leu Trp Glu Thr Thr Thr Gly Arg Cys Leu
   595 600 605
   Arg Thr Phe Phe Gly His Leu Glu Gly Val Trp Ala Leu Gly Ala Asp
   610 615 620
   Thr Leu Arg Ile Val Ser Gly Ala Glu Asp Arg Met Val Lys Ile Trp
   625 630 635 640
   Asp Pro Arg Thr Gly Lys Cys Glu Arg Thr Phe Thr Gly His Ser Gly
   645 650 655
   Pro Val Thr Cys Ile Gly Leu Gly Asp Ser Arg Phe Ala Thr Gly Ser
   660 665 670
   Glu Asp Cys Glu Val Arg Met Tyr Ser Phe Arg Asn
   675 680
<210> 7
   <211> 10
   <212> DNA
   <213> Artificial Sequence
<220>
   <223> Description of Artificial Sequence:Primer DNA
<400> 7
   accgcygcya gcgcycgrtt 10
<210> 8
   <211> 18
   <212> DNA
   <213> Artifical Sequence
<220>
   <223> Description of Artificial Sequence:Primer DNA
<400> 8
   sgakccrtgy ttctcrgt 18
<210> 9
   <211> 8
   <212> DNA
   <213> Artificial Sequence
<220>
   <223> Description of Artificial Sequence:Adapter DNA
<400> 9
   acctgccc 8
<210> 10
   <211> 44
   <212> DNA
   <213> Artificial Sequence
<220>
   <223> Description of Artificial Sequence:Adapter DNA
<400> 10
   ctaatacgac tcactatagg gctcgagcgg ccgcccgggc aggt 44
<210> 11
   <211> 22
   <212> DNA
   <213> Artificial Sequence
<220>
   <223> Description of Artificial Sequence:Primer DNA
<400> 11
   ctaatacgac tcactatagg gc 22
<210> 12
   <211> 26
   <212> DNA
   <213> Artificial Sequence
<220>
   <223> Description of Artificial Sequence:Primer DNA
<400> 12
   ttttccatct ccagctgggc tacgcg 26
<210> 13
   <211> 28
   <212> DNA
   <213> Artificial Sequence
<220>
   <223> Description of Artificial Sequence:Primer DNA
<400> 13
   agggatctgc gtgctgtact tggtgtgt 28
<210> 14
   <211> 24
   <212> DNA
   <213> Artificial Sequence
<220>
   <223> Description of Artificial Sequence:Primer DNA
<400> 14
   tggaacggac agtgcgagag acta 24
<210> 15
   <211> 27
   <212> DNA
   <213> Artificial Sequence
<220>
   <223> Description of Artificial Sequence:Primer DNA
<400> 15
   atgtcagatg agcacatcgc tcgtcag 27
<210> 16
   <211> 25
   <212> DNA
   <213> Artificial Sequence
<220>
   <223> Description of Artificial Sequence:Primer DNA
<400> 16
   ctagttatcg gtgcccacac ccttc 25
<210> 17
   <211> 18
   <212> DNA
   <213> Artificial Sequence
<220>
   <223> Description of Artificial Sequence:Primer DNA
<400> 17
   cgratgtgyg aacaacac 18
<210> 18
   <211> 21
   <212> DNA
   <213> Artificial Sequence
<220>
   <223> Description of Artificial Sequence:Primer DNA
<400> 18
   caamccctcs aggtgmccga a 21
<210> 19
   <211> 26
   <212> DNA
   <213> Artificial Sequence
<220>
   <223> Description of Artificial Sequence:Primer DNA
<400> 19
   aggaagcccc cagccacatt tcttgc 26
<210> 20
   <211> 22
   <212> DNA
   <213> Artificial Sequence
<220>
   <223> Description of Artificial Sequence:Primer DNA
<400> 20
   acgattcttg cctcagcctc cg 22
<210> 21
   <211> 26
   <212> DNA
   <213> Artificial Sequence
<220>
   <223> Description of Artificial Sequence:Primer DNA
<400> 21
   atggcccaac cgaccggaga acttac 26
<210> 22
   <211> 26
   <212> DNA
<213> Artificial Sequence
<220>
   <223> Description of Artificial Sequence:Primer DNA
<400> 22
   ttaattgcgg aaactgtaca tgcgca 26
<210> 23
   <211> 30
   <212> DNA
   <213> Artificial Sequence
<220>
   <223> Description of Artificial Sequence:Primer DNA
<400> 23
   gaaggagata tacatatgga ttacgaccag 30
<210> 24
   <211> 30
   <212> DNA
   <213> Artificial Sequence
<220>
   <223> Description of Artificial Sequence:Primer DNA
<400> 24
   cccccgggag ctcctagtta tcggtgccca 30
<210> 25
   <211> 30
   <212> DNA
   <213> Artificial Sequence
<220>
   <223> Description of Artificial Sequence:probe DNA
<400> 25
   ggacgactgc taccacggta gcgcgcgggc 30
<210> 26
   <211> 30
   <212> DNA
   <213> Artificial Sequence
<220>
   <223> Description of Artificial Sequence:probe DNA
<400> 26
   gcccgcgcgc taccgtggta gcagtcgtcc 30
<210> 27
<211> 27
   <212> DNA
   <213> Artificial Sequence
<220>
   <223> Description of Artificial Sequence:Primer DNA
<400> 27
   caagcttcc aatatgtcag atgagca 27
<210> 28
   <211> 26
   <212> DNA
   <213> Artificial Sequence
<220>
   <223> Description of Artificial Sequence:Primer DNA
<400> 28
   aagctctaga tacacaaggt aacaga 26
<210> 29
   <211> 24
   <212> DNA
   <213> Artificial Sequence
<220>
   <223> Description of Artificial Sequence:Primer DNA
<400> 29
   gagcggataa caatttcaca cagg 24